# EUROPEAN PATENT SPECIFICATION

(11) **EP 4 754 533 B1**
(45) Date of publication and mention of the grant of the patent: **25.11.2026**
(21) Application number: 24790393.3
(22) Date of filing: 11.10.2024
(51) Int. Cl.: G01N 33/68

(54) **METHODS FOR ASSESSING PROTEIN ABSORPTION FROM THE GUT**
VERFAHREN ZUR BEURTEILUNG DER PROTEINABSORPTION AUS DEM DARM
MÉTHODES D'ÉVALUATION DE L'ABSORPTION DES PROTÉINES PAR L'INTESTIN

(30) Priority: 26.10.2023 SE 2330478; 16.11.2023 SE 2330513; 20.03.2024 SE 2430141; 21.03.2024 SE 2430143
(43) Date of publication of application: 10.06.2026
(73) Proprietor: Anara AB, 231 32 Trelleborg (SE)
(72) Inventor: PIERZYNOWSKI, Stefan, 231 32 Trelleborg (SE); PIERZYNOWSKA, Kateryna, 231 32 Trelleborg (SE)
(74) Representative: Novitas Patent AB
(86) International application number: PCT/EP2024/078732
(87) International publication number: WO 2025/087708

(56) References cited:
- BERNARD L. ET AL: "Effect of the methodology on peptide amino acid concentrations in blood and plasma of sheep", ARCHIV FUER TIERERNAEHRUNG - ARCHIVES OF ANIMAL NUTRITION., vol. 54, no. 4, 1 December 2001 (2001-12-01), DE, pages 281 - 296, XP093222642, ISSN: 0003-942X, DOI: 10.1080/17450390109381985
- R�RAT A. ET AL: "Comparaison de deux techniques d'estimation (ninhydrine vs TNBS) de l'azote des acides amin�s circulants, appliqu�es � l'�tude de l'absorption intestinale de solutions d'acides amin�s libres ou de petits peptides", REPRODUCTION, NUTRITION, DEVELOPMENT, vol. 27, no. 5, 1 January 1987 (1987-01-01), FR, pages 955 - 966, XP093222688, ISSN: 0181-1916, DOI: 10.1051/rnd:19870708
- CORTE ZORAIDA ET AL: "Biological variation of free plasma amino acids in healthy individuals", CLINICAL CHEMISTRY AND LABORATORY MEDICINE, vol. 48, no. 1, 25 November 2009 (2009-11-25), DE, pages 99 - 104, XP093214401, ISSN: 1434-6621, DOI: 10.1515/CCLM.2010.008
- MENDEL FRIEDMAN: "Applications of the Ninhydrin Reaction for Analysis of Amino Acids, Peptides, and Proteins to Agricultural and Biomedical Sciences", JOURNAL OF AGRICULTURAL AND FOOD CHEMISTRY, vol. 52, no. 3, 1 February 2004 (2004-02-01), pages 385 - 406, XP055009377, ISSN: 0021-8561, DOI: 10.1021/jf030490p
- PIERZYNOWSKA KATERYNA ET AL: "Modified throughput ninhydrin method for the qualitative assessment of dietary protein absorption in pig plasma", BIOLOGY METHODS AND PROTOCOLS, vol. 9, no. 1, 25 October 2024 (2024-10-25), England, XP093222720, ISSN: 2396-8923, Retrieved from the Internet <URL:https://academic.oup.com/biomethods/article-pdf/9/1/bpae078/60500613/bpae078.pdf> DOI: 10.1093/biomethods/bpae078

## Description

### TECHNICAL FIELD

The present invention relates to methods for assessing protein absorption from the gut, diagnosis of protein malabsorption as such or together with insufficiency of gut proteolytic enzymes including exocrine pancreatic insufficiency (true and apparent insufficiency).

### BACKGROUND TO THE INVENTION

The digestion of dietary proteins can be regarded to begin in the acidic pH conditions of the stomach through the action of pepsin. The product of pepsin action is polypeptide fragments of high molecular weight, traditionally named proteoses and peptones (Kyle et al., 2022).

Peptones are further digested in the small intestine by gut erapsin - peptidases complex including pancreatic proteases (trypsin, chymotrypsin, elastase, carboxypeptidases and gut peptidases), which process peptones and endogenous protein directly and in plastein reaction (Northtrop, 1947) into free amino acids and peptides containing between 2-8 amino acids. The ratio of plasma peptides to free amino acids is estimated to be about 4:1. According latest papers from the field, about 80% of dietary protein is absorbed in the form of peptides (Shen and Matsui, 2019).

Protein maldigestion and malabsorption occurs when pancreatic proteases are not secreted into the gut due to different reasons e.g., bariatric surgery (Han et al, 2021) -> apparent insufficiency where enzymes entering intestine are not synchronized with digesta appearance, cystic fibrosis in children and elderly in which exocrine pancreas insufficiency is common (Price et al, 1977; Rothenbacher et al, 2005, Löhr et al, 2018) -> true insufficiency where the pancreas is not producing enzymes. The protein malabsorption (as well as fat malabsorption) is treated with exogenous pancreatic enzyme replacement therapy (PERTs) (Fieker et al, 2011).

There are methodological problems in performing a quick assessment of protein maldigestion and PERT efficacy on protein digestion. Historically, the coefficients of fat and nitrogen absorption (CFA and CNA, respectively) measurement has been used for regulatory approval of porcine-derived PERT (Gan et al, 2017; Borowitz et al, 2022; Freedman et al 2023). These methods have known limitations being indirect methods with poor reliability, sensitivity and onerous requirements for long lasting stool collection. Method based on HPLC, GC, MS or their combinations for measurement of amino acids in the blood absorbed from the diet (free and total after hydrolysis) are time consuming, expensive but do not give consistent results (Bernard L, Chauveau B, Rémond D. Effect of the methodology on peptide amino acid concentrations in blood and plasma of sheep. Arch Tierernahr. 2001;54(4):281-96. doi: 10.1080/17450390109381985. PMID: 11921851).

There is a long-felt need for improved tests of protein absorption, rather than protein excretion in stool, which are inexpensive, fast and easy to perform. Thus, an object of the present invention is the provision of alternative and/or improved methods for assessing protein absorption from the gut of an individual, and applications for such methods for purposes such as treating exocrine pancreatic insufficiency, assessing biological age, assessing quality of dietary protein and predicting efficacy and absorption of certain drugs, among others.

### DEFINITIONS

The term "comprising" is to be interpreted as including, but not being limited to.

Numerical values in the specification and claims of this application should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 to 10" is inclusive of the endpoints, 2 and 10, and all the intermediate values).

The term "about" can be used to include any numerical value that can vary without changing the basic function of that value. When used with a range, "about" also discloses the range defined by the absolute values of the two endpoints, e.g., "about 2 to about 4" also discloses the range "from 2 to 4." The term "about" may refer to plus or minus 10% of the indicated number.

The term **"exocrine pancreatic insufficiency"** (abbreviated **EPI**) refers to a condition where the amount of pancreatic digestive enzymes secreted by the pancreas to the small intestine is reduced to the degree that it affects digestion. In the small intestine, the pancreatic digestive enzymes play a major role in digestion of carbohydrates, proteins, and fats, and may also have hormone-like functions. The reduction may be due to reduced production of pancreatic digestive enzymes, a blockage in pancreatic ducts preventing the pancreatic digestive enzymes from reaching the small intestine, or from problems in pancreatic regulation (for instance, the timing of enzymatic secretion is out of phase with introduction of digesta to the duodenum). EPI can occur in several health conditions including pancreatitis, cystic fibrosis, pancreatic cancer, type 1 diabetes or type 2 diabetes, inflammatory bowel disease, celiac disease, surgery of the pancreas or upper gastrointestinal tract including bariatric surgery, Sjögren's syndrome, alcohol abuse and growth retardation. EPI can also result from degenerative processes related to aging. Common symptoms of EPI include malnutrition, weight loss or inability to gain weight, and diarrhoea.

**Pancreatic enzyme replacement therapy (PERT)** is the standard treatment of EPI and involves oral administration of digestive enzymes such as pancrelipase, suitably formulated (typically encapsulated) to survive passage through the stomach to the small intestine. Brand names containing porcine pancrelipase include Creon^{™} and Pancreon^{™}. As an alternative to porcine pancrelipase, a microbial-derived pancrelipase is marketed as Ultresa^{™}. In the context of the present invention, any therapy providing replacement of even a single type of a pancreatic digestive enzyme is considered a PERT. Preferred form of PERT in the context of the present invention involves administering a suitable protease.

The term **postprandial** in the present context refers to a period of 0-8 h after a meal, preferably 0-4h after a meal, more preferably 0-3h after a meal, and most preferably 0-2h after a meal.

### BRIEF DESCRIPTION OF THE FIGURES

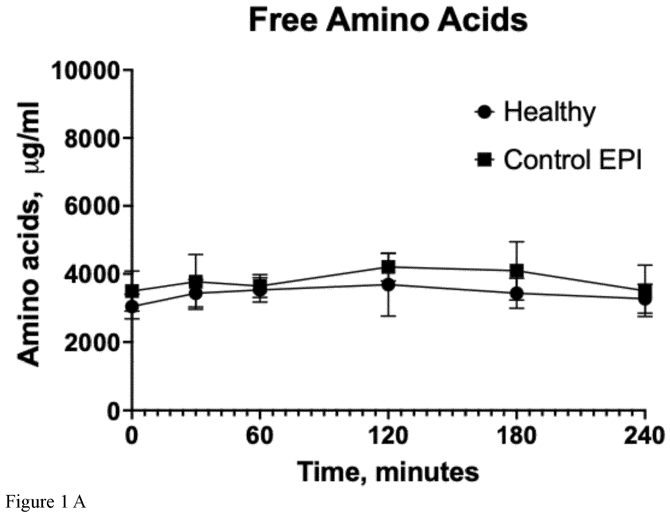

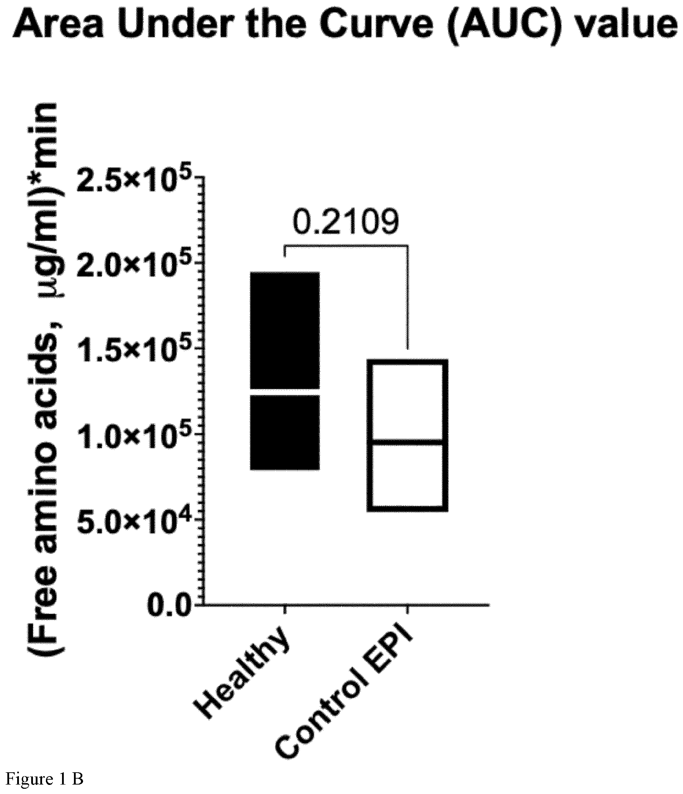

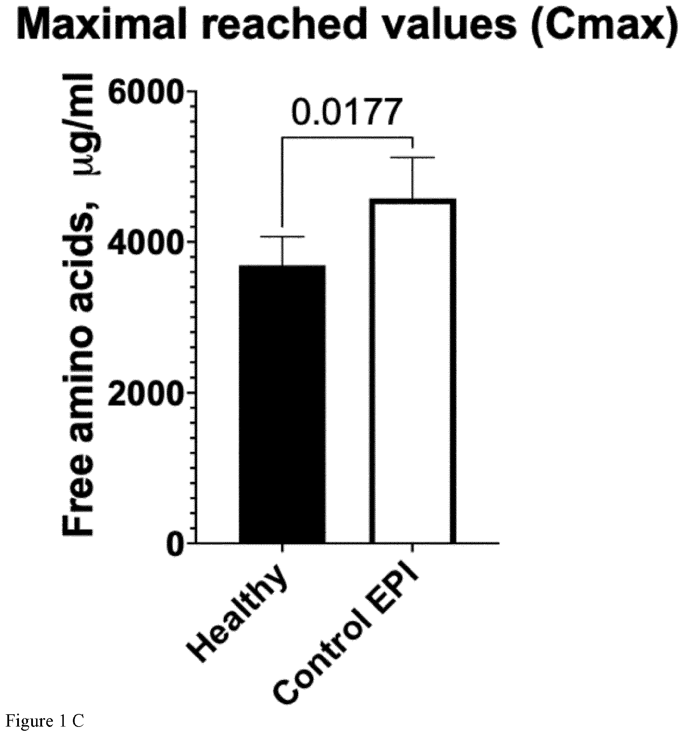

**Figure 1****. Levels of free amino acids in porcine plasma.** Healthy - the group of intact animals (n=6), Control EPI - group of animals with exocrine pancreatic insufficiency (n=6). Data on AUC is presented with line at the Mean and Cₘₐₓ is presented as Median±IQR, data on amino acid levels on separate timepoints is given as Mean±SD. Differences were considered significant if p≤0.05; differences were considered as a trend when p≤0.1. P values are given with the results bars.

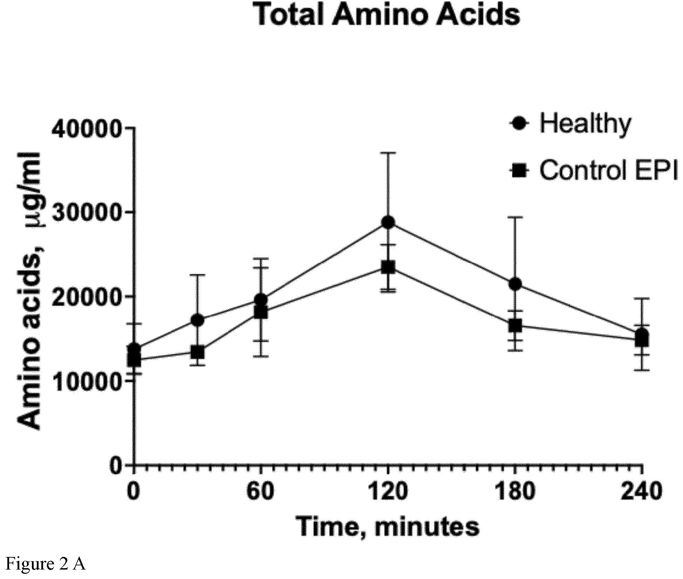

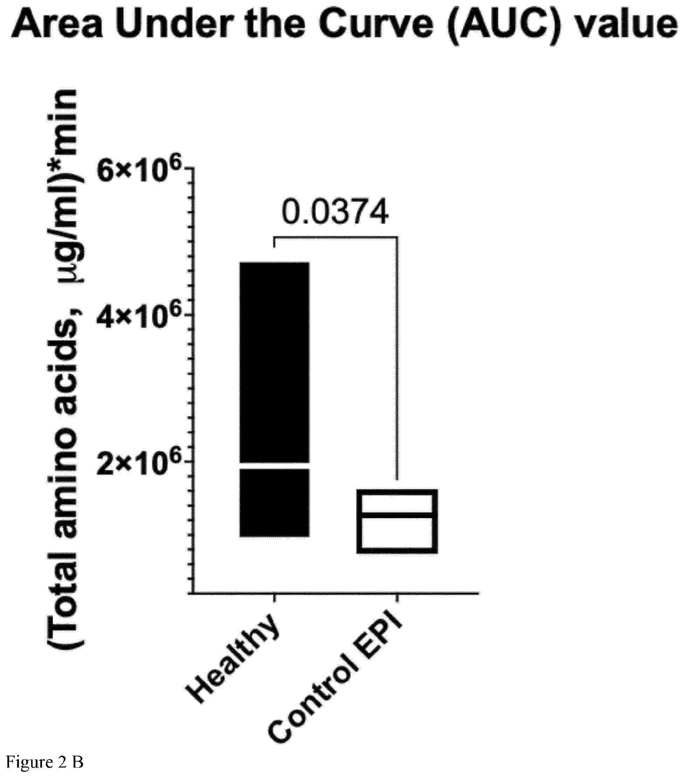

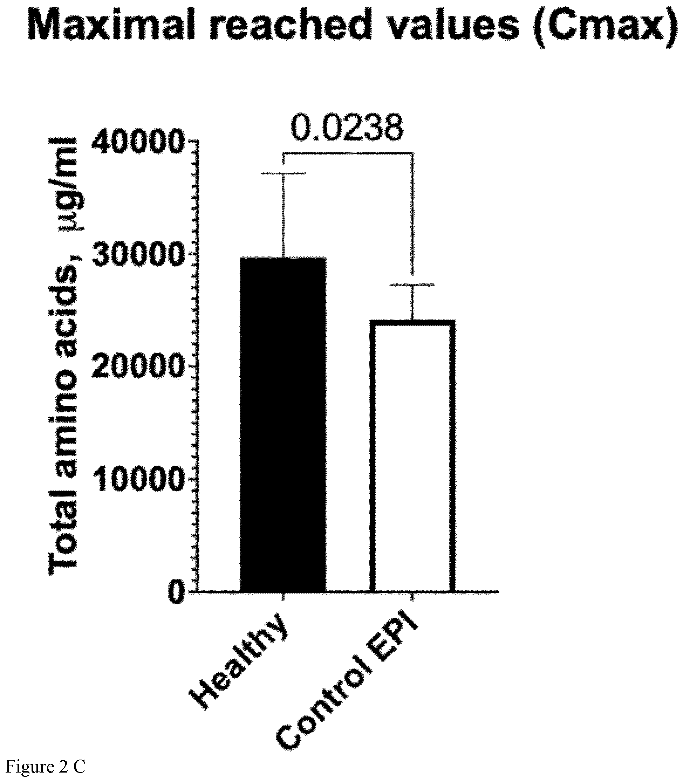

**Figure 2****. Levels of total amino acids in porcine plasma.** Healthy - the group of intact animals (n=6), Control EPI - group of animals with exocrine pancreatic insufficiency (n=6). Data on AUC is presented with line at Mean, Cₘₐₓ and data on amino acid levels on separate timepoints is given as Mean±SD. Differences were considered significant if p≤0.05; differences were considered as a trend when p≤0.1. P values are given with the results bars.

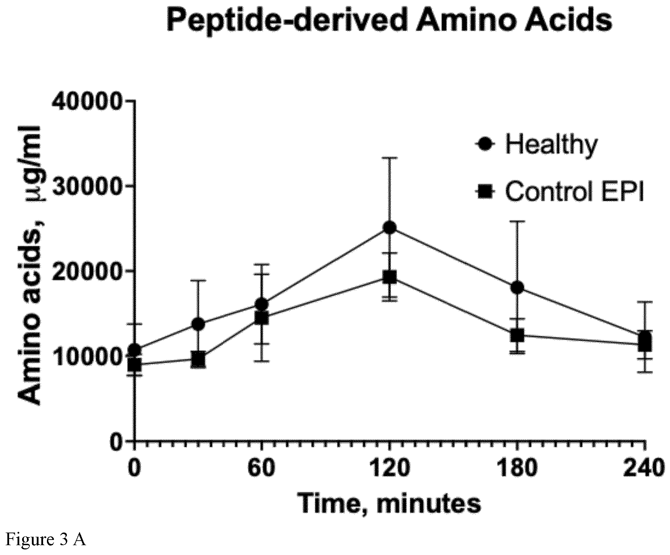

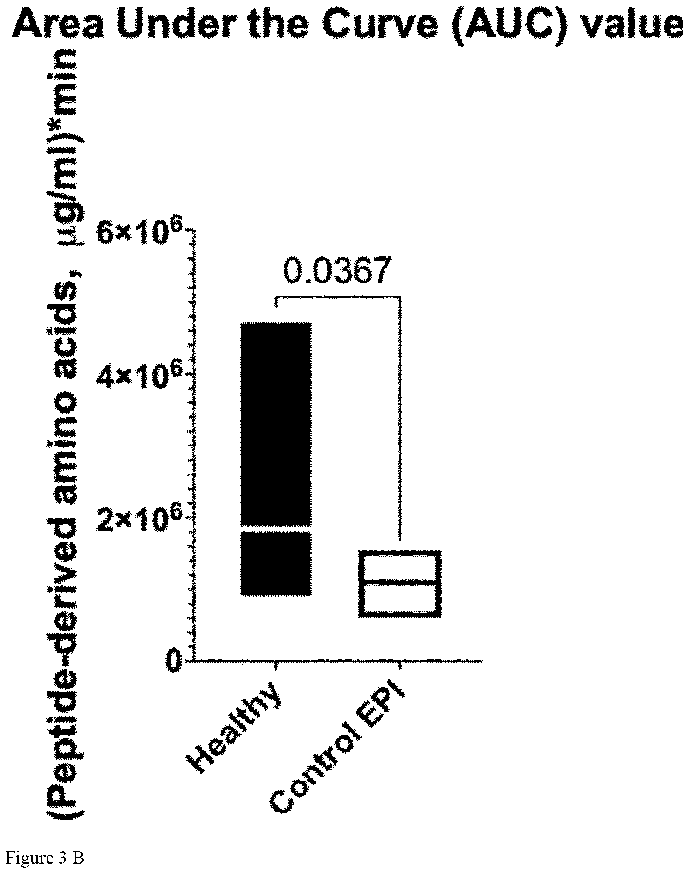

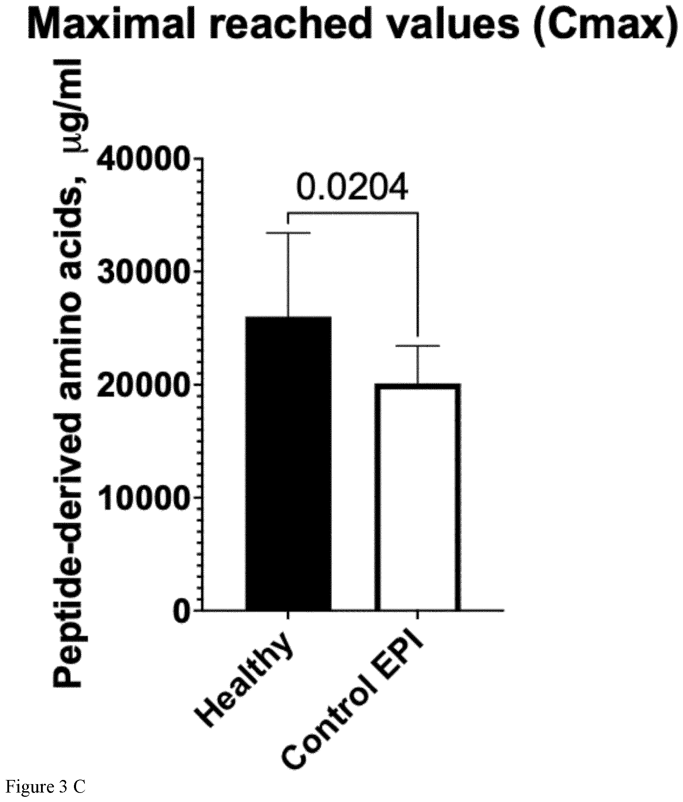

**Figure 3****. Levels of peptide-derived amino acids in porcine plasma.** Healthy - the group of intact animals (n=6), Control EPI - group of animals with exocrine pancreatic insufficiency (n=6). Data on AUC is presented with line at Mean, Cₘₐₓ and data on amino acid levels on separate timepoints is given as Mean±SD. Differences were considered significant if p≤0.05; differences were considered as a trend when p≤0.1. P values are given with the results bars.

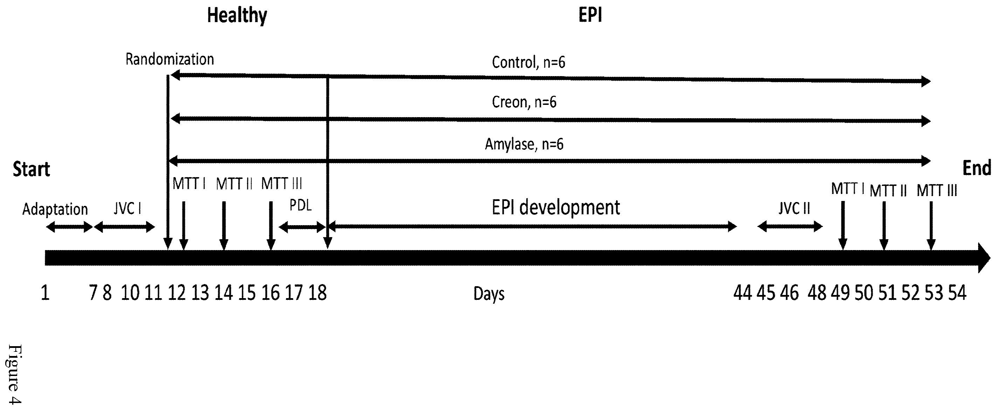

**Figure 4****. Experiment design and study treatments for PERT efficacy comparison.** JVC - jugular vein catheterization surgery; RMTT - regular meal tolerance test (at figure nominated as MTT); PDL - pancreatic duct ligation surgery; EPI - exocrine pancreatic insufficiency.

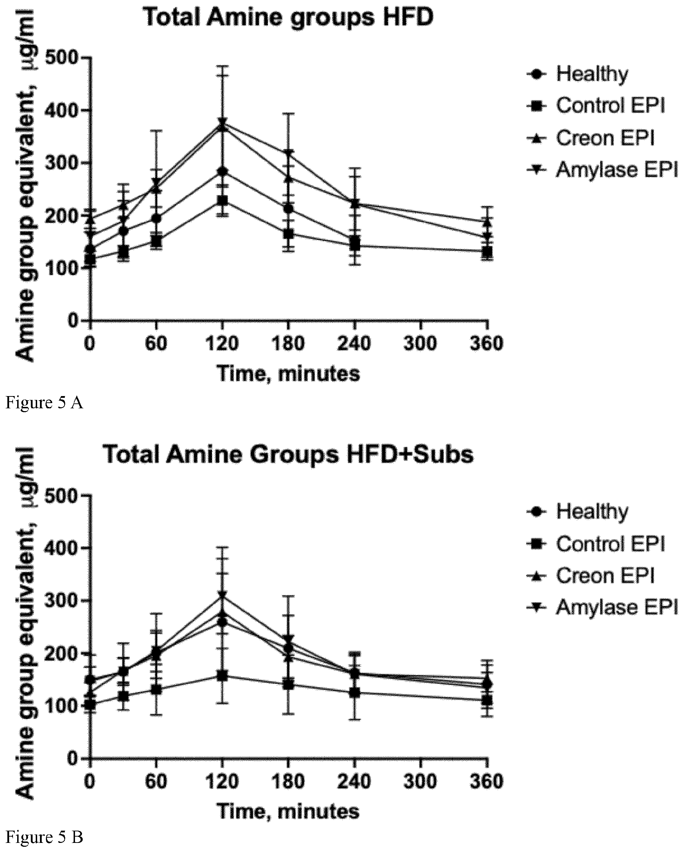

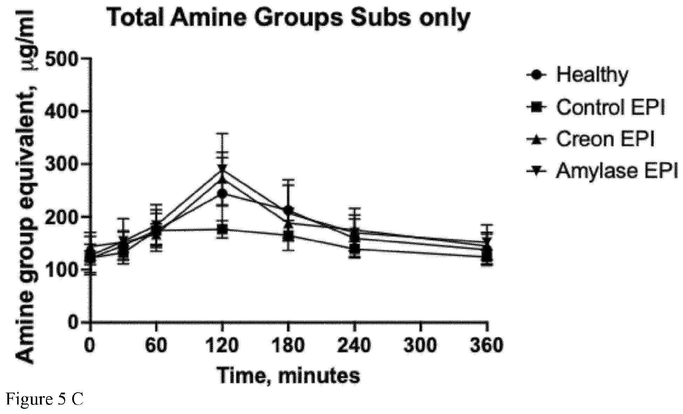

**Figure 5****. Postprandial change in plasma levels of Total Amine Groups.** Data is presented for Regular Meal Tolerance Test (RMTT) performed with HFD, HFD+SUB; SUB in Control Healthy and EPI pigs, as well as in EPI pigs treated with Creon or Amylase. Data is expressed as mean ± standard deviation (± SD).

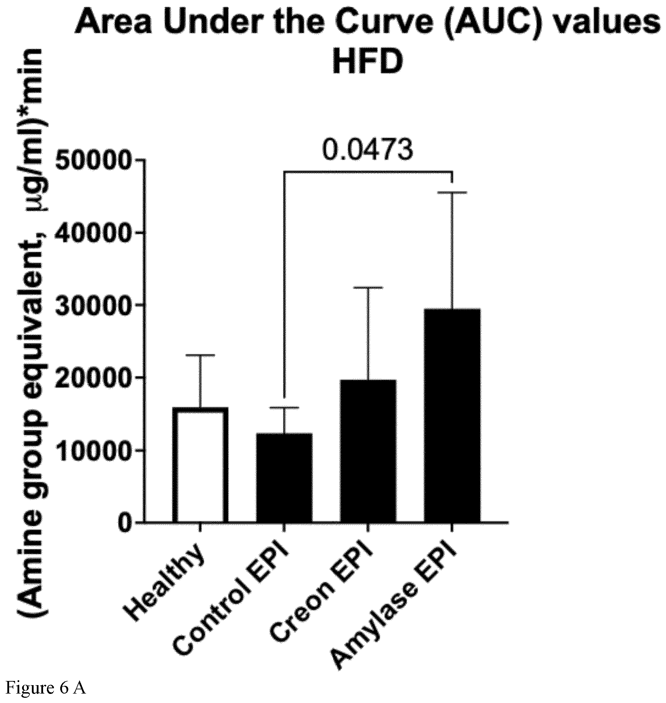

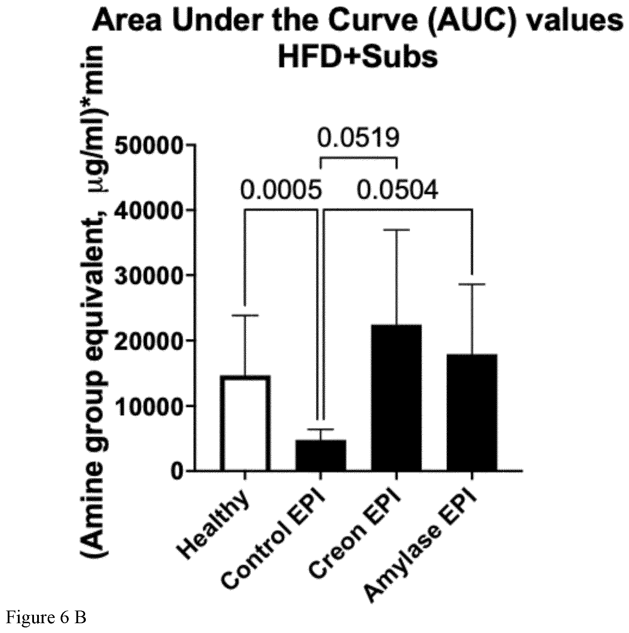

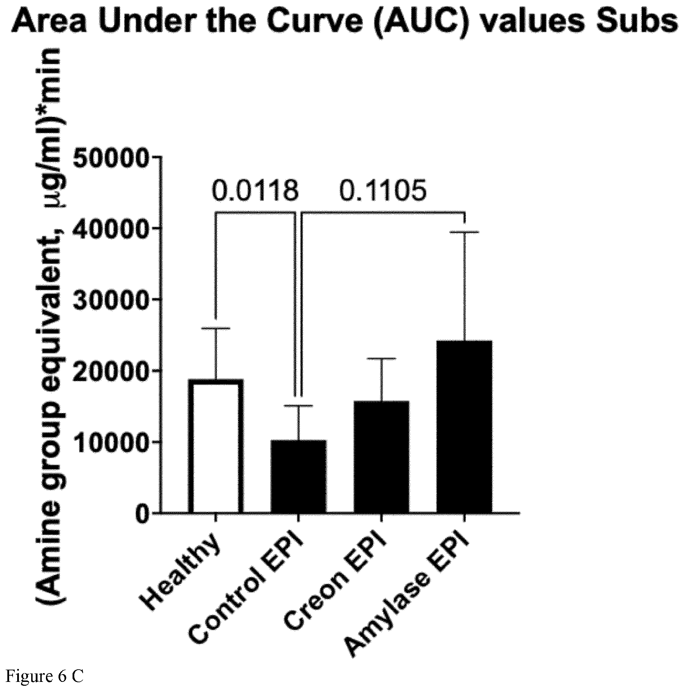

**Figure 6****. Absorption Area under the Curve (AUCs) of Total Amine Groups.** Data is presented for Regular Meal Tolerance Test (RMTT) performed with HFD, HFD+SUB; SUB in Control Healthy and EPI pigs, as well as in EPI pigs treated with Creon or Amylase. Data is expressed as mean ± standard deviation (± SD). AUC data is baseline adjusted. The differences between the results were considered significant when p<0.05. p-values ranging between 0.1 and 0.05 were considered as a trend. p-values are given with the result bars.

### SUMMARY OF THE INVENTION

The inventors have developed a high-throughput colorimetric/fluorometric method for assessing protein absorption from the gut (see Examples 1-3). Surprisingly, the method was sufficiently sensitive to detect both free amino-acids and peptides absorbed from the gut to the blood, with only a simple molecular weight-based separation of resident plasma proteins.

Six healthy pigs prior and after EPI development were used in the applicability test. Large plasma proteins were removed with centrifugal filters with cut off of 10 kDa molecular weight. Free and total amino acids were estimated with ninhydrin method in the filter permeate before and after its hydrolysis. Peptide-derived (mainly from nutritional dipeptides and tripeptides) amino acids were quantified by subtracting free amino acids from total amino acids. Accuracy of the analyse was close to 100%, precision - expressed as percentage intermediate precision was below 5%, linearity - r²=0.9914.

In brief, the inventive method can serve a tool to monitor of protein digestion and absorption, as a superior alternative to the indirect, stool-based technique of coefficient of nitrogen absorption (can) measurement. It is also cost effective and does not require advanced instrumentation, compared to HPLC, GC, MS or the like. The inventive method is precise, reproducible, and can also be used easily in the clinical or research settings. Data obtained from the method can be used to assess situations where pancreatic enzymes (or deficiency thereof) affect the absorption ratio of amino acids vs. peptides of dietary protein absorption (see Example 4).
a. The present invention is set out in the appended independent claims. Preferred embodiments are set out in dependent claims and the following description.

The arrangement of the present disclosure into sections with headings and subheadings is merely to improve legibility and is not to be interpreted limiting in any way, in particular, the division does not in any way preclude or limit combining features under different headings and subheadings with each other.

### DETAILED DESCRIPTION

**Method for assessing protein absorption** The first aspect defines the invention in its general sense. Other aspects make use of the method defined by the first aspect.

In a first aspect, the present invention provides an in vitro high throughput method for assessing protein absorption from the gut of a subject, comprising:
a. Providing a blood sample from the subject;
b. Substantially removing proteins having size over the size limit 40 kDa from the sample to provide a deproteinized sample;
c. With a colorimetric ninhydrin assay determining the level of free amino acids in a first subsample of the deproteinized sample, the level being designated CF;
d. Subjecting a second subsample of the deproteinized sample to hydrolysis converting any remaining polypeptides and peptides to free amino acids, thus obtaining a hydrolyzed sample;
e. With a colorimetric ninhydrin assay determining the level of free amino acids in the hydrolyzed sample, the level being designated CT;
f. Optionally, calculating the level of peptide-derived amino-acids in the blood sample, the level being designated CP, wherein CP=CT-CF; and
g. Assessing protein absorption from the gut of the subject based on the values of CF and CT, and optionally further using CP.

The blood sample may be a plasma sample, a serum sample, a whole haemolyzed blood sample or isolated, haemolyzed red blood cell sample. The sample is preferably a plasma or serum sample, most preferably a plasma sample.

The determination is colorimetric, and involves the ninhydrin reaction, which is a classical method for amino-acid quantification. Commercial reagents utilizing various chemistries are available for colorimetric (e.g using genipin) or fluorometric determinations of free amino acids in a sample.

Ninhydrin is a powerful oxidizing agent and, in its presence, the amine group in amino acids, peptides and proteins undergoes oxidative deamination, liberating ammonia, CO₂, a corresponding aldehyde and the reduced form of ninhydrin, hydrindantin. The colorimetric assay is based on that two molecules of ninhydrin (2,2- dihydroxyindane-1,3-dione) subsequently react with a free alpha-amino acid to produce a deep purple or blue colour, known as Ruhemann's purple (Ruhemann, 1910). In this reaction, ninhydrin acts as an oxidizing agent and causes the deamination and decarboxylation of amino acids at an elevated temperature. The reaction is then followed by condensation between the reduced ninhydrin molecules and released ammonia and the second molecule of n-hydrindantin. By the end of the reaction, a diketohydrin complex is formed, which has a deep purple colour. For amino acids like proline and hydroxyproline, this test instead yields an iminium salt, which is yellow-orange in colour. Similarly, proteins with a free amine group like asparagine react with the ninhydrin reagent to form a brown-coloured product. Amino acids from hydrolysed protein or peptides also react with ninhydrin (Mendel, 2004). Ninhydrin reagents can be conveniently obtained from commercial sources.

The amount of the coloured complex formed is proportional to the concentration of amino acids in the solution to be analysed due to the presence of the amine group (NH₂). The intensity of the Ruhemann's purple colour can be measured spectrophotometrically, preferably at a wavelength of about 570 nm. Alternatively, or additionally, the intensity of the yellow-orange colour resulting from the reaction with proline/hydroxyproline can be measured at a wavelength of about 440 nm. To measure both types of amino acids simultaneously, 520 nm wavelength can be used.

The hydrolysis step (d) preferably involves acid hydrolysis, usually at an elevated temperature. Exposure to an acid at elevated temperature, e.g. acetic acid buffer for 1 h at 100°C can be used to release the amino acids from peptides, thus enabling measurement of the sum of free and peptide-derived amino acids (i.e. total amino-acids). Naturally, many different methods of performing peptide hydrolysis are known in the art.

### Removal of interfering blood proteins

Most of the proteins present in the blood sample (such as serum/plasma/haemolyzed red blood cells/haemolyzed blood) are much larger (e.g. albumin about 67 kDa, immunoglobulins about 160 kDa, fibrinogen about 340 kDa), than the dietary peptides which are mostly 2-3, occasionally up to 8 amino acids (i.e. roughly 200-800 Da) that are the target of the measurements. It is therefore possible to eliminate colorimetric/fluorometric (e.g. ninhydrin) assay interference from these proteins by removal techniques based on molecular size, such as ultrafiltration. Other suitable methods for protein removal include selective precipitation (e.g. using a denaturing solvent or trichloroacetic acid at a concentration selectively precipitating large proteins), gel filtration and dialysis.

Preferably, the size limit of removal is about 30 kDa, more preferably about 20 kDa, even more preferably about 10 kDa, yet more preferably about 5 kDa, still more preferably about 3 kDa, most preferably about 1 kDa.

The protein removal in step b is such that it preferably results in at least 98% reduction of proteins having molecular weight over the size limit, preferably at least 99% reduction, more preferably at least 99.9% reduction.

The protein removal in step b preferably involves ultrafiltration, preferably with a filter with the desired molecular weight size limit, for example a 10 kDa cut off filter. Suitable filters working with centrifugal force or vacuum suction are well known in the art.

### Conclusions from the assessment

An increased postprandial level of CF relative to postprandial level of CT or CP compared to a (healthy) reference is indicative of protein malabsorption and/or exocrine pancreatic insufficiency. This is because in malabsorption, the dietary peptides are not properly absorbed, with their absence leading to free amino-acid domination in circulation.

A reduced postprandial level of CP compared to a (healthy) reference is indicative of protein malabsorption and/or exocrine pancreatic insufficiency.

The results may be used in evaluating the effects of bariatric surgery in a patient, preferably by comparing the values before and after surgery. Suitably, the subject is given a similar protein meal challenge at before and after surgery prior to sampling for the inventive assay. Reduced CT or CP after surgery is indicative of malabsorption.

The result can also be used for evaluating exercise and training efficacy, preferably comparing the value of CP and CP of the different meals in time points after before and after training.

The reference may be a mean value of comparably measured values in a set of reference samples from a set of reference subjects free of protein malabsorption and/or exocrine pancreatic insufficiency. Said set may comprise at least 10 reference subjects. The test subject is preferably matched with the reference subjects in terms of age and/or sex.

Alternatively, the reference may be comparably measured values in a sample from the same subject taken at a different point in time, for instance in the case when values before and after bariatric surgery are compared to evaluate the effects of the bariatric surgery, or in the case when values before and after exercise are compared to evaluate the effects of physical training.

### Treatments

The present text discloses (but does not claim) a method of treatment for exocrine pancreatic insufficiency (including elderly insufficiency, neonatal insufficiency, alcohol abuse related insufficiency, bariatric surgery related pancreatic enzyme insufficiency and pepsin insufficiency) in a subject in need thereof, comprising:
a. assessing protein absorption using the method of the first aspect in a subject suspected of having exocrine pancreatic insufficiency; and
b. If protein malabsorption is detected, administering oral pancreatic enzyme replacement therapy (PERT) to the subject.

The present text also discloses (but does not claim) a method of treatment for exocrine pancreatic insufficiency in a subject in need thereof, comprising:
a. assessing protein absorption using the method of the first aspect in a subject having been diagnosed with exocrine pancreatic insufficiency and receiving PERT; and
b. if protein malabsorption is detected, increasing the dosage of the PERT administered the subject.

The subject in referred to above may have undergone bariatric surgery.

The PERT may comprise orally administering a protease to the subject. The PERT preferably comprises orally administering pancrelipase (which includes a protease) to the subject. The pancrelipase may be animal/ porcine-derived or microbial-derived.

### Assessing the quality of a dietary protein by assessing protein absorption from the gut

In a second aspect of the present invention, there is provided an in vitro high throughput method for assessing the quality of a dietary protein by assessing protein absorption from the gut, comprising:
a. Feeding the dietary protein to be assessed (test dietary protein) to a test subject;
b. Providing a blood sample taken from the subject after feeding;
c. Substantially removing proteins having size over the size limit of 40 kDa from the sample to provide a deproteinized sample;
d. With a colorimetric ninhydrin assay determining the level of free amino acids in a subsample of the deproteinized sample, the level being designated CF;
e. Subjecting a subsample of the deproteinized sample to hydrolysis converting any remaining polypeptides and peptides to free amino acids, thus obtaining a hydrolyzed sample;
f. With a colorimetric ninhydrin assay determining the level of free amino acids in the hydrolyzed sample, the level being designated CT;
g. Optionally, calculating the level of peptide-derived amino-acids in the blood sample, the level being designated CP, wherein CP=CT-CF;
h. Assessing protein absorption from the gut of the subject based on the values of CF and CT, and optionally further using CP; and
i. Assessing the quality of the dietary protein based on the results from assessment of protein absorption.

The steps b-h are performed as described for the first aspect. The blood sample may be as described for the first aspect.

The blood sample is preferably taken 1h, 2h, 3h, 4h and/or 6h after the feeding took place.

If CP or CT is low compared to CP or CT (respectively) of a reference dietary protein of satisfactory dietary quality measured in similar manner, this indicates that the test dietary protein is difficult to digest, and the quality of the test dietary protein is deemed poor. By "low" in this context is meant less than 50%, or in order of increasing preference, less than 40%, 30%, 20%, 10%, 5% or 1%.

Conversely, if CP or CT is similar or high compared to CP or CT (respectively) of a reference dietary protein of satisfactory dietary quality measured in similar manner, this indicates that the test dietary protein is digestible, and the quality of the test dietary protein is deemed satisfactory. Using CP is preferred, but since CF is relatively constant, the assessment can also be made using CT directly without calculating CP. By "high" in this context is meant at least 50%, or in order of increasing preference, at least 60%, 70%, 80%, 85%, 90%, 95% or 100%.

The test dietary protein may be from a conventional or preferably a non-conventional source, such as microbial, fungal, insect or synthetic. The reference dietary protein can be any protein source of known quality, including meat protein, blood albumin, yolk protein, cereal protein, milk-derived protein or protein from a vegetable source. The tested source of protein may be tested by itself and/or as a component of mixed meal (containing additional macro- and micronutrients apart from the tested protein).

The test subject may be a laboratory or farm animal such as a mouse, rat, gerbil, guinea pig, rabbit, chicken, turkey, pig, cat or dog, or a human being.

### Determining the biological age of a subject

In a third aspect, there is provided an in vitro high throughputmethod for determining the biological age of a subject, comprising:
a) Providing a blood sample from the subject;
b) Substantially removing proteins having size over the size limit of 40 kDa from the sample to provide a deproteinized sample;
c) With a colorimetric ninhydrin assay determining the level of free amino acids in a subsample of the deproteinized sample, the level being designated CF;
d) Subjecting a subsample of the deproteinized sample to hydrolysis converting any remaining polypeptides and peptides to free amino acids, thus obtaining a hydrolyzed sample;
e) With a colorimetric ninhydrin assay determining the level of free amino acids in the hydrolyzed sample, the level being designated CT;
f) Optionally, calculating the level of peptide-derived amino-acids in the blood sample, the level being designated CP, wherein CP=CT-CF;
g) Assessing protein absorption from the gut of the subject based on the values of CF and CT, optionally further using CP; and
h) Estimating the biological age of the subject based on the assessed level of protein absorption, optionally based on CP, and further independently optionally in combination with other physiological indicators of biological age.

The steps a-g are performed according to the first aspect. The blood sample may be as described for the first aspect.

The biological age estimation may include comparing the assessed level of protein absorption to a reference value representative of similarly assessed protein absorption in a group of reference subjects being representative of subjects of a particular age or age interval, or a set of such reference values. The reference subject(s) may be matched with the subject in terms of age, sex and/or health status.

The reference values may be average or median (preferably median) protein absorption assessed according to the first aspect for: 1/ 100 individuals 6 - 12 years of age , 2/ 100 individuals 13 - 21 years, 3/ 100 individuals 21 - 30 years, 4/ 100 individuals 31- 45 years, 5/ 100 individuals 46 - 60 years, 6/ 100 individuals 61-75 years, 7/ 100 individuals 76 -80 years, 8/100 individuals older than 81 years. The subject's measured protein absorption may then be compared to the reference value obtained from the age group which is closest the subject's chronological age.

The biological age estimation can be based on measurements of additional indicators of biological age, such as DNA methylation, telomere length, proteomics analysis, glycomics analysis, clinical biomarkers, wearable sensor data and/or other known markers for biological age.

In certain instances, CP is calculated, and the biological age estimation is performed based on CP.

### Assessment of drug absorption and efficacy

In a fourth aspect, there is provided an in vitro high throughputmethod for assessing the capacity of drug absorption from the gut in a subject and/or drug efficacy in a subject, comprising:
a) Providing a blood sample from the subject;
b) Substantially removing proteins having size over the size limit of 40 kDa from the sample to provide a deproteinized sample;
c) With a colorimetric ninhydrin assay determining the level of free amino acids in a subsample of the deproteinized sample, the level being designated CF;
d) Subjecting a subsample of the deproteinized sample to hydrolysis converting any remaining polypeptides and peptides to free amino acids, thus obtaining a hydrolyzed sample;
e) With a colorimetric ninhydrin assay determining the level of free amino acids in the hydrolyzed sample, the level being designated CT;
f) Optionally, calculating the level of peptide-derived amino-acids in the blood sample, the level being designated CP, wherein CP=CT-CF;
g) Assessing protein absorption from the gut of the subject based on the values of CF and CT, and optionally further using CP; and
h) Assessing drug absorption capacity and/or expected drug efficacy of the subject based on the assessment of protein absorption.

The steps a-g are performed according to the first aspect. The blood sample may be as described for the first aspect.

If the protein absorption is lower than average for the individual, the drug absorption capacity is also deemed lower than average ted for the individual. Conversely, if the protein absorption is higher than average for the individual, the drug absorption capacity is also deemed higher than average for the individual. The assessment may include comparing the assessed protein absorption to a reference value obtained from a reference subject or a set of reference subjects. The reference subject(s) may be matched with the individual in terms of age, sex and/or health status. Expected drug efficacy can be estimated based on the assessed drug absorption. The dosing of the drug to the subject may be adjusted according to the results.

The assessed drug absorption may peptide transporter (PepT1)-dependent or amino-acid transporter-dependent. However, the method is not limited to drugs having a particular transport mechanism, as long as the absorption correlates with amino acid absorption and/ or di- and tripeptide absorption. Examples of drugs that may be assessed by the method include bestatin, β-lactam antibiotics, angiotensin-converting enzyme (ACE) inhibitor, valacyclovir, L-DOPA-L-Phe and levodopa.

In some instances, CP is calculated, and the drug uptake assessment is performed based on CP. This is advantageous for drugs using the same uptake mechanism as di- and tripeptides. In such cases, the free amino acid level does not provide additional information and may instead add noise.

In a related fifth aspect, the individual drug dosing regimen can be devised or adjusted based on the results obtained from the method of the sixth aspect. The seventh aspect provides a method for calculating an individual dosing regimen of a drug for a subject, comprising:
a. Assessing the capacity of drug absorption from the gut of the subject using the method of the fourth aspect; and
b. Calculating an individual drug dosing regimen for the subject based on the assessment.

The calculated individual dosing regimen can then be applied to clinical practice by a physician. For individuals where the peptide absorption measured is lower than average, a higher-than-average dose is administered, thus achieving effective plasma concentrations for the treatment. For individuals where the peptide absorption is higher than average, a lower-than-average dose is administered, thus avoiding excess exposure from the drug and consequently minimizing side effects.

The drug is preferably transported by PepT1 or an amino-acid transporters. The drug may be an anticancer agent, bestatin, β-lactam antibiotics, angiotensin-converting enzyme (ACE) inhibitor, valacyclovir, L-DOPA-L-Phe or levodopa.

### Additional applications

In further aspects, the present invention relates to a method of optimizing diet for a subject, based on testing the subject's protein uptake after ingesting various foodstuffs using the method of the first aspect. The various foodstuffs are compared against each other based on the relative protein uptake in the subject. The subject's diet is adjusted to include or increase the proportion of foodstuffs for which the observed protein uptake is relatively good, and to avoid or decrease the proportion of foodstuffs with relatively poor uptake. The subject can be for instance an elderly/geriatric patient, a patient with protein malabsorption, a child with growth retardation or an athlete.

The present invention also relates to a method of optimizing a training regimen for a subject such as an athlete, based on testing the subject's protein uptake after different training exercises whose effects on protein uptake are compared to each other. Again, this would use the first aspect of the invention. The subject's training regimen is then adjusted to include or increase the relative volume of exercises after which the observed protein uptake is relatively higher in the training regimen, and to avoid or decrease the relative amount of exercises after which the observed protein uptake is relatively lower in the training regimen.

The present invention also relates to a method of screening productive farm animals for productive individuals, by screening farm animals for their protein uptake capacity using the method of the first aspect. Individual animals having higher than average protein uptake capacity can be selected for production, and the individual animals showing poor protein uptake can be culled.

### Discussion of the results in Examples

Based on results obtained from healthy volunteers and healthy animals and then from the same animals after development of exocrine pancreatic insufficiency the inventors have unexpectedly demonstrated that the ninhydrin method is sensitive enough to quickly detect levels of peptides/derived amino acids in blood, and clearly shows differences between mode of protein absorption in healthy vs. EPI pigs (Fig. 1, 2, and 3) and Examples Surprisingly, levels of free amino acids in blood of healthy and EPI pigs do not react to feeding. Moreover, the maximal level (Cₘₐₓ) of free amino acids is significantly (p<0.05) higher in EPI pigs when compared to healthy animals. Calculations show that levels of free amino acids as compared to total amino acids in Healthy and EPI pigs account for about 10% and 20% respectively. Conversely, total amino acids levels (which are mainly composed of peptide derived amino acids) are significantly higher in Healthy pigs when compared to the EPI animals.

A possible explanation of above-described features may involve plastein rection in the gut (Watanabe et al, 1992; Gong et al, 2015). The enzyme (proteinase)-catalyzed formation of peptides and polypeptides from amino acids and peptides of a stomach hydrolysate i.e., the formation of peptide bonds, is called the plastein reaction. Plastein reaction taking place in the gut lumen starts in duodenum (or possibly in the stomach after pancreatic enzyme physiological reflux) when peptone created by pepsine in the stomach appears and is mixed with pancreatic and gut peptidases.

Thus, pancreatic enzymes not only cleave proteins but also digest peptides derived by pepsin (non-specific peptidase) activity in the stomach. Pancreatic peptidases modify end-products of all protein digestion by specific endopeptidase, carboxypeptidases and dipeptidase by coupling the free amino acids to peptides, peptides to peptides, etc. in plastein reaction. The lack of pancreatic peptidases and, thus, a slowed down plastein reaction in gut lumen could explain the higher level of free amino acids and lower level of peptide/derived amino acids in blood of EPI animals. It is postulated that the same ratio and post-prandial changes pattern of free and total amino acids would be seen in aged patients as exocrine pancreas function is decreased during aging (Löhr et al, 2018).

All mentioned above provoke the considerations about current standard of care and actual treatment of cystic fibrosis patients of different age in the same way as the elderly patients with EPI being treated with the same doses of pancrelipase. PERT drugs currently present on market aim mainly to improve fat absorption. It appears that requirements of EPI patients can differ much, thus PERT drugs usage (formulations) should be reconsidered and adapted to age and type of sickness, etc. For instance, growth of the cystic fibrosis patient is probably more dependent on absorbed peptides than on fat, while glucose homeostasis and diabetes type II/III development is mainly dependent on alpha-amylase levels in circulation (Pierzynowski et al, 2023). Further studies on role of absorbed peptides vs. amino acids in disease should be undoubtfully conducted. Studies from 90s (Pierzynowski et al, 1997) it can be hypothesized that blood peptides are used for tissue protein synthesis while absorbed amino acids for protein reparation.

Most of the dietary protein is absorbed in the form of peptides (Goodman, 2010), as opposed to free amino acids. Measurement of peptides, as opposed to free amino acids, may be an important marker of health in addition to assessing protein absorption. Absorbed peptides are expected to play a key role in the regulation of body metabolism and growth (Pierzynowski et al 1997). Thus, over and above the delivery of nutrients, peptides can induce numerous physiological effects (Miner - Williams et al, 2014; Shen and Matsui, 2019; Choi et al, 2012; O'Keeffe et al, 2018 2-5; Pellegrini et al., 2004). Peptides are more efficiently absorbed when compared to free amino acids (Webb, 1986). The exact mechanisms by which peptides enter the blood stream are not fully elucidated. It is well documented that di- and tripeptides pass directly from the gut lumen to the blood, via their transporter (PepT 1) found on enterocytes (Vang et al, 2017). Furthermore, it is suggested that the quantitative and qualitative composition of pancreatic endopeptidases affect the profile (quantity and quality) of peptides (Pierzynowski et al., 2005). Having a high-throughput inexpensive tool to measure peptide absorption may help advance this field of study.

The results of the current study indicate that the modified, high-throughput, ninhydrin method is a reliable method, which can be used for the measurement of pre- and postprandial free amino acids and dietary-derived peptides. Both accuracy and precision reflect how close a measurement is to an actual value. More specifically, accuracy reflects how close a measurement is to a known or accepted value, while precision reflects how reproducible measurements are, even if they are far from the actual value. Verification results for the modified ninhydrin method, shown in Table 1 are satisfactory for the primary parameters measured: accuracy, precision (repeatability and intermediate precision). Linearity is the ability to provide laboratory test results that are directly proportional to the concentration of the quantity to be measured in a test sample. We have demonstrated that this high throughput, modified, ninhydrin method is highly linear. These factors are essential for a test that can be used in clinical research and in general clinical applications. The CNA measurement has never been shown to have these critical characteristics.

In summary, herein is provided a fast, reliable, and simple method for the measurement of peptide-derived amino acids, which can serve as direct biomarkers of protein digestion and absorption and a superior alternative to the indirect, stool-based CNA measurement. This the inventive method is reproducible and physiologically relevant and can also be used easily in the clinical or research settings, as proven on EPI pig's model.

### REFERENCES

Kyle JS, Matthew D. Wilcox, Peter I. Chater, lain A. Brownlee, Maria I. Zakhour, Katherine M. R. M. Banecki, Jeffery P. Pearson. Pepsin properties, structure, and its accurate measurement: a narrative review. Ann Esophagus 2022;5:31 | http://dx.doi.org/10.21037/aoe-20-9.
Northrop JH. Plastein from pepsin and trypsin. J Gen Physiol. 1947 May 20; 30(5): 377-378. Doi: 10.1085/igp.30.5.377
Shen W. and Matsui T. Intestinal absorption of small peptides: a review. International Journal of Food Science and Technology 2019, 54, 1942-1948. Doi:10.1111/ijfs.14048.
Price HL, Gazzard BG, Dawson AM. Steatorrhea in the elderly. British Medical Journal, 1977, 1, 1582-1584)
Ming-Lun Han, Jyh-Ming Liou , Kong-Han Ser, Jung-Chien Chen, Shu-Chun Chen, Wei-Jei Lee. Changes of serum pepsinogen level and ABC classification after bariatric surgery. Journal of the Formosan Medical Association 120 (2021) 1377e1385, /doi.org/10.1016/j.jfma.2020.10.029
Rothenbacher D, Low M, Hardt PD, Klo R H-U, Ziegler H, and Btenner H. Prevalence and determinants of exocrine pancreatic insufficiency among older adults: Results of a population-based study. Scandinavian Journal of Gastroenterology, 2005; 40: 697 /704.
Löhr J-M, PanicN, Vujasinovic M, and Verbeke CS. The ageing pancreas: a systematic review of the evidenceand analysis of the consequences Intern Med. 2018;283:446-460. Doi: 10.1111/joim.12745.
Aaron Fieker A, Philpott J, Armand M. Enzyme replacement therapy for pancreatic insufficiency: present and future. Clinical and Experimental Gastroenterology 2011:4 55-73. DOI: 10.2147/CEG.S17634.
Gan C, Chen Y-H, Liu L, Gao J-H, Tong H, Tang Ch-W, and Liu R. Efficacy and safety of pancreatic enzyme replacement therapy on exocrine pancreatic insufficiency: a meta-analysis. Oncotarget. 2017 Nov 7; 8(55): 94920-94931. Doi:10.18632/oncotarget.21659.
Borowitz D, Aronoff N, Cummings LC, Maqbool A, and Mulberg AE. | Coefficient of Fat Absorption to Measure the Efficacy of Pancreatic Enzyme Replacement Therapy in People With Cystic Fibrosis. Gold Standard or Coal Standard? Pancreas 2022;51: 310-318. DOI: 10.1097/MPA.0000000000002016.
Freedman SD, Zaworski K, Pierzynowska, Pierzynowski S, Gallotto R, Sathe M, Borovitz D. Validation of an omega-3 substrate challenge absorption test as an indicator of global fat lipolysis. PloS ONE 2023, 18(5): e0284651. https:// doi.org/10.1371/journal.pone.0284651.
Ruhemann, S. Triketohydrindene hydrate. Trans. Chem. Soc.1910, 97, 2025-2031. Mendel F. Applications of the Ninhydrin Reaction for Analysis of Amino Acids, Peptides, and Proteins to Agricultural and Biomedical Sciences J. Agric. Food Chem. 52, 385-406 38, 2004.
Watanabe M & Soichi A. The plastein Reaction: Fundamentals and Applications, Biochemistry of Food Proteins. 271-305, 1992.
Gong M, Mohan A, Gibson A, Udenigwe ChC. Mechanisms of plastein formation, and prospective food and nutraceutical applications of the peptide aggregates. Biotechnology Reports 5 (2015) 63-69. Doi.org/10.1016/j.btre.2014.12.003.
Pierzynowski SG, Stier Ch, Pierzynowska K. A hypothesis that Alpha-amylase Evokes Regulatory Mechanisms Originating in the Pancreas, Gut and Circulation, which Govern Glucose/Insulin Homeostasis. World J Diabetes. 2023 September 15; 14(9): 1341-1348, DOI: 10.4239/wjd.v14.i9.1341.
Pierzynowski S, Puchala R, Sahlu T. 1997. Effect of dipeptide administered to a perfused area of skin in angora goats. Journal of Animal Science. 75. 3052-3056. Goodman BE. 2010. Insights into digestion and absorption of major nutrients in humans. Adv. Physiol. Educ.. 34. 44-53.
Miner-Williams WM, Stevens BR, Moughan PJ. Are intact peptides absorbed from the healthy gut in the adult human?. Nutr. Res. Rev. 2014, 27(2). 308-329.
Choi J, Sabikhi L, Hassan A, Anand S. 2012. Bioactive peptides in dairy products. Int. J. Dairy Technol.. 65. 1-12.
O'Keeffe MB, FitzGerald RJ. Whey protein hydrolysate induced modulation of endothelial cell gene expression. J. Funct. Foods. 2018. 40. 102 109.
Pellegrini A, Andreas J, Hulsmeierb W, Hunziker P, Thomas U. Proteolytic fragments of ovalbumin display antimicrobial activity. Biochimica et Biophysica Acta 1672: 76- 85. 2004.
Webb KE, Jr. Amino acid and peptide absorption from the gastrointestinal tract . Fed Proc. 1986 Jul;45(8):2268-71.
Wang Chun-Yang, Shu Liu, Xiao-Nv Xie, Zhi-Rong Tan. Regulation profile of the intestinal peptide transporter 1 (PepT1). Drug Design, Development and Therapy:11, 3511-3517, 2017.
Pierzynowski SG, Kruszewska D, Weström B. -The quality of the dietary protein digestion affects animal performance and regulates gut bacteria growth - hypotheses and facts. In: Biology of the growing Animals, Series Editors: Pierzynowski SG, Zabileski R.,Volume Editors: Biology of the Nutrition in Growing Animals, Editors: Mosenthin R, Zentek J, Zebrowska T. 120-143, Elsevier, 2005.
Chapman, I.; Oberoi, A.; Giezenaar, C.; Soenen, S. Rational Use of Protein Supplements in the Elderly-Relevance of Gastrointestinal Mechanisms. Nutrients 2021, 13, 1227.

### EXAMPLES

The following examples are not to be regarded as limiting. For further information on the experimental details, the skilled reader is directed to a separate section titled Materials and Methods.

### Example 1: Estimation of free, total, and peptide derived amino acids with ninhydrin method

The main modification of the original ninhydrin method was the development of a high-throughput method on microplate. Centrifugal filters of 10 kDa were employed to separate free amino acids and peptides from large plasma proteins e.g., albumin and fibrinogen.

The intact permeate was used to estimate the levels of free amino acids, while the hydrolysed permeate was used to estimate total amino acids. Peptide-derived amino acids were quantified by subtraction of free amino acids from total amino acids: Peptide-derived amino acids = Total amino acids - Free amino acids

### Reagents required

1. Standard amino acid stock solution (Amino acids standard, cat# AAS18, Supelco, Sigma Aldrich ) 260 ul +740 dH2O.
2. 0.2 M Acetate buffer (pH=5.5) (0.84 g NaOH + 1.465 ml glacial acetic acid + 100 ml dH2O)
3. 8% w/v of Ninhydrin reagent (N4876, Sigma-Aldrich) [ Preparation: Weigh 8g of ninhydrin and dissolve in 100ml of 99.8% acetone]. Light sensitive! Should be used within 15 minutes.
4. 50% v/v ethanol.
5. Distilled water.

### Apparatus and glassware required

1. 10 kDa spin columns (Amicon Ultra-0.5 Centrifugal Filter Unit, cat # UFC5010, Millipore, Sigma Aldrich).
2. Test / Boiling tubes (1.5 ml tubes with screw caps).
3. Pipettes [micropipette].
4. Microplate with flat bottom
5. Water bath or dry heating block.
6. Spectrophotometer

### Standard curve preparation

An amino acid stock solution was prepared. Various volumes (20-70 µl) of the standard amino acid stock were pipetted into appropriately labelled tubes to achieve 6 standard concentrations of 30.0-105.0 µg / ml.

### Sample preparation

350 µl of separated plasma was transferred to a centrifuge column (10 kDa spin columns) and centrifuged at 14.000 g for 30 minutes at room temperature. No significant differences in amino acid content between separated serum or plasma samples were noted. Samples with high (105 µg/ml), medium (60 µg/ml) and low (30 µg/ml) amino acid concentrations were prepared for use in the verification test.

### Ninhydrin assay

Different volumes (20-70 ul) of standard amino acid stock solution were transferred to the respective labelled test tubes to reach standard concentrations of between 30-105 µg/ml. The test samples were then transferred to test tubes: a) for free amino acids, 30 ul of centrifugate was transferred, b) for total amino acids, 30 ul of acetate buffer was added to 30 ul of centrifugate and the liquid was then brought to the boil in a water bath for 1 hour for hydrolysis. After that, 30 ul of hydrolysate was taken and transferred to the new test tube.

To standardize the signal intensity, 15 ul of acetic buffer was added to all standards and free amino acid samples. The volume in the test tubes was then made up to 1000 ul, using distilled water. Test tubes labelled 'blank' were filled with 15 ul of acetic buffer and 985 ul of distilled water. Finally, 250 ul of ninhydrin reagent was added to all the test tubes and the tubes were then gently vortexed and placed in a boiling water bath for 15 minutes. After cooling the test tubes on ice, 250 ul of 50% ethanol was added to each test tube and mixed well. 300 ul of the reaction mixture from the respective test tubes was then transferred to a microplate and the absorbance was recorded at 570 nm, using a spectrophotometer. Results were adjusted for dilution.

### Calculation of free amino acids

${C}_{final free} = \left(CSD*33.3\right) / 1.1$

Where C_{final} is the final concentration of free amino acids, ug/ml; CSD - concentration of free amino acids obtained from the standard curve; 33.3 - dilution factor for 30 ul of sample and 1.1 is the coefficient compensating for the plasma deproteinization.

### Calculation of total amino acids

${C}_{final total} = \left(CSD*2*33.3\right) / 1.1$

Where C_{final} is the final concentration of total amino acids, ug/ml; CSD - concentration of free amino acids obtained from the standard curve; 2 - dilution factor for hydrolysis; 33.3 - dilution factor for 30 ul of hydrolyzed sample and 1.1 is the coefficient compensating for the plasma deproteinization.

### Calculation of peptide derived amino acids

${C}_{final peptide derived} = {C}_{final total} - {C}_{final free}$

### Example 2: Validation of the high-throughput ninhydrin method

Following quantification, the following parameters were evaluated: Accuracy, Precision (Repeatability and Intermediate Precision) and Linearity.

### Accuracy

Accuracy was determined based on the percentage recovery of amino acids from the high, medium and low amino acid concentration samples. All samples were run in triplicate. Percentage (%) recovery at each level was determined according to the equation below. $% Recovery = \frac{Measured conc . of peptides}{Theoretical conc . of peptides} \times 100$

Results from the accuracy analysis are shown in Table 1.

### Target acceptance criteria

Accepted percentage recovery should be between 90 - 110 %. We obtained the following percentage recovery: 106.2%, 96.1% and 97.7% for the low, medium and high amino acid concentration samples, respectively (Table 1).

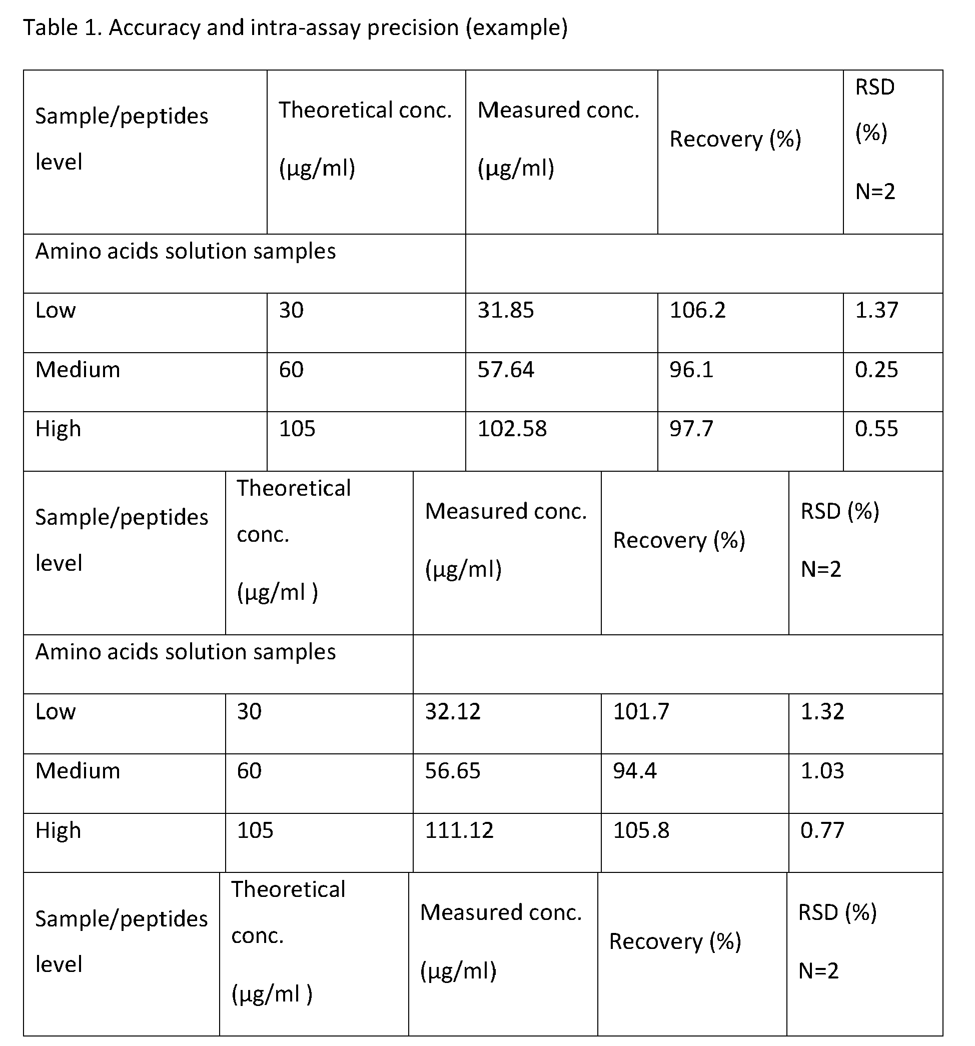

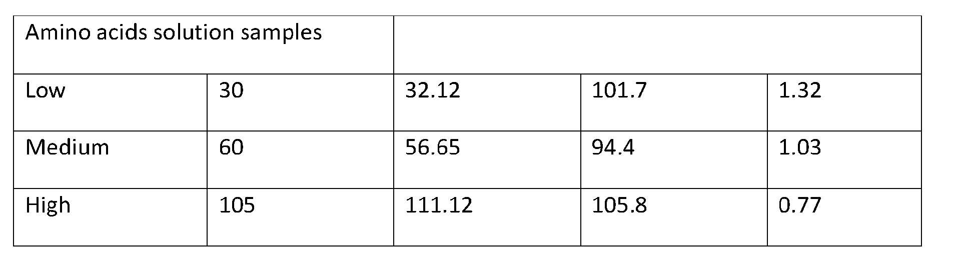

### Precision

Precision expresses the closeness of agreement between a series of measurements obtained from multiple sampling of the same homogeneous sample, under the prescribed conditions. It is usually expressed as percentage Relative Standard Deviation (%RSD).

### Repeatability or intra-assay precision

Repeatability expresses the precision under the same operated conditions over a short interval of time. Nine determinants covering the specified range for the procedure (e.g. 3 concentrations/2 replicates each) were assessed. To calculate repeatability, we used the data obtained for three samples with high, medium and low concentrations of amino acids (Table 1).

### Target acceptance criteria

The RSD (n=3) at each level and the overall RSD should be ≤ 5 %. Reported as the average of the RSDs (n=3) for the three amino acid levels, repeatability for the 3 amino acid solution samples was calculated as 0.72% [(1.37+0.25 +0.55)/3= 0.72 %] (Table 1).

### Intermediate precision

Intermediate precision expresses within-laboratory variations: different days, different analysts, different equipment. Etc. In order to assess intermediate precision, samples with high, medium and low concentrations of amino acids were re-analysed on three different days. Samples were analysed in duplicates.

### Target acceptance criteria

The RSD at each level and the overall RSD should be ≤ 5 %. Intermediate precision for amino acid solutions were 3.91%, 3.00% and 2.52% for the low, medium and high amino acid concentration levels, respectively. The overall RSD for all 3 amino levels was 3.14 % (Table 2).

**Table 2. Intermediate precision data (example)**

| Amino acids content level | Mean concentration (n=2) | | | %RSD | Overall %RSD |
|---|---|---|---|---|---|
| | Day 1 | Day 2 | Day 3 | | |
| Low (30 µg/ml ) | 35.44 | 33.95 | 32.78 | 3.91 | |
| Medium (60 µg/ml) | 53.25 | 55.14 | 56.54 | 3.00 | 3.14 |
| High (105 µg/ml) | 101.67 | 102.9 | 106.68 | 2.52 | |

### Recovery

Plasma samples were spiked with certain level of free amino acids (500-2500 µg/ml) prior to centrifugation and the recovery rates were calculated by comparing the measured value to the amount of free amino acids in unspiked samples. Average recovery level was 81% with range from 69 to 93%.

### Standard curve fit

To obtain a standard curve, 6 standards of the amino acid mixture and a blank were used. The standard curve (cubic) was then plotted as the amino acid concentration (µg/ml) of the respective standard vs. the obtained value of optical density measured at a wavelength of 570 nm. The coefficient of determination (*r*²) was then calculated.

### Target acceptance criteria

The *r²* values for each of the standard curves should be ≥ 0.98 (Table 3).

**Table 3. Coefficient of determination (r²) values for standard curves on different days**

| | Day 1 | Day 2 | Day 3 | Mean *r²* |
|---|---|---|---|---|
| *r²* | 0.9946 | 0.9873 | 0.9924 | 0.9914 |

### Example 3: Proof of concept study in pig model of EPI

Results on the levels of free amino acids in porcine plasma are given in the Figure 1 A-C. The levels of free amino acids in Healthy animals are about 2000 µg/ml, while EPI animals have free amino acids concentrations about 4000 µg/ml . The observed difference is even reflected both in increased AUC and Cₘₐₓ. In both groups of experimental animals, the levels of free amino acids are stable and do not change significantly after meal.

Results for the levels of peptide derived + free amino acids described as total amino acids in porcine plasma are given in the Figure 2 A-C, while on Fig. 3 A-C. The initial levels of total amino acids or peptide derived amino acids in both Healthy and EPI pigs are similar are about 13 000 vs. 10 000 µg/ml respectively. Both total and peptide derived amino acids increased after feed (Fig 2 A and Fig 3 A). In contrast to free amino acid alone, total amino and peptide derived amino acids after a meal increased significantly. However, healthy animals achieved significantly higher levels of total and peptide derived amino acids up to three hours after meal, with Tmax at 120 min after feeding. The differences between groups are reflected both in AUC and Cₘₐₓ values which are significantly higher in the Healthy group when compared to the EPI one (Figure 2 B, C and Fig 3 B, C).

### Example 4: Evaluation of the impact of digestive enzyme supplementation on protein uptake

The impact of exogenous digestive enzymes supplementation on dietary protein digestion and nutritional peptide vs. amino acids absorption was tested during different type of regular meal tolerance tests (RMTT) on eighteen male pigs before and after exocrine pancreatic insufficiency (EPI) development using adapted ninhydrin method of the present invention (see Fig 4 for experimental outline).

The types of macronutrients used in the isoenergetic RMTT test meal affect the percentual relation between absorbed di-, tripeptides and free amino acids. Absorption of di- and tripeptides acids was lower in EPI pigs as compared to values obtained before pancreatic duct ligation. Addition of Creon or Amylase to the meal during the RMTT significantly and similarly increased absorption of di- and tripeptides. Interestingly, expression of PepT1 in the duodenum and jejunum of EPI pigs was lower than that in healthy pigs and was restored to healthy values by enzyme (Creon or Amylase alone) supplementation.

In summary, the adapted ninhydrin method allow with good probability to estimate percentual rate for nutritional di-, tripeptide and free amino acids postprandial absorption from dietary protein as well as on their inter-digestive percentual composition in blood. Exogenous microbial Amylase and Creon essentially and similarly improve postprandial peptide absorption as well as expression of PepT1. Thus, age related PepT1 disappearance and limited peptide absorption can be related to exocrine pancreatic insufficiency in the elderly.

**Table 4. Composition of Regular Meal Tolerance Test (RMTT) meals, which accounted for 1% of the daily requirements of pigs weighing approximately 16 kg. The meals were isoenergetic.**

| | RMTT I | RMTT II | RMTT II |
|---|---|---|---|
| High Fat Diet (HFD) | 28 g prot. cereales + 32 g fat - raps oil + 79 g starch cereals + 6 g crude fiber + 8 g ash + 7 g water = 160 g | 18 g prot. cereales+ 20 g rapes oil + 50 g starch cereals + 3 g crude fiber+ 4 g ash + 5 g water = 100 g | 0 |
| SUBSTRATE (SUB) | 0 | 4 g DHA/EPA + 10 g whey + 32 g potatoe starch + 8 g soya oil 3 g ash + 3 water = 60 g | 4 g DHA/EPA + 28 g whey + 85 g potatoe starch + 28 g soya oil 8 g ash + 7 water = 160 g |

### Total (amino acids + dipeptides + tripeptides) quantitative absorption calculation

The effects of Creon and Amylase supplementation on plasma levels of total amine groups during the RMTTs are presented in figures 5 A-C. Plasma levels of free amine groups did not change postprandially.

EPI pigs exhibited a significantly lower total amino acids absorption range than that of Control Healthy pigs, when HFD was enriched with substrates or substrates were given alone. (Fig. 6 A-C).

### Total quantitative protein absorption

Composition of isoenergetic feed containing similar level of different proteins and other macro components of different origin affects proteolysis and absorption of its end products. These results go in line with previously obtained data on EPI pigs fed with identical diets where the dependence of carbohydrate absorption from its dietary source was confirmed. Thus, we could reveal and emphasize the hidden differences in the protein digestion and further absorption using the different source of dietary protein. Our present study also confirms data obtained by Rohm et al (2020) where the clear dependence of peptide absorption from the dietary protein source was shown in human beings.

### Example 5: Assessment of biological age of a subject

Peptide absorption measurement using the method of the invention is performed for reference groups of healthy 20 individuals with ages of i) 20-30, ii) 31-40), iii) 41-50, iv) 51-60, v) 61-70, and vi) 71-80. Similarly, peptide absorption is measured in test subjects using the method of the invention. By comparison to the averages reference groups, the test subjects' biological ages are assessed as falling to the age group having the closest average. When the assessed biological age is lower than the test subject's chronological age, it is considered that at least in terms of gut health, the test subject is in better than average condition. A biological age corresponding to the chronological age is indicative of average condition for the test subject. When the assessed biological age is higher than the test subject's chronological age, it is considered that at least in terms of gut health, the test subject is in poorer than average condition. Appropriate interventions are proposed for the test subject.

### Example 6: Drug dose adjustment

Peptide absorption measurement using the method of the invention is performed for individuals that require treatment with a cephalosporin. For individuals where the peptide absorption measured is lower than average, a higher-than-average dose of cephalosporin is administered, thus achieving effective plasma concentrations. For individuals where the peptide absorption is higher than average, a lower-than-average dose of cephalosporin is administered, thus avoiding excess exposure.

### Example 7: Diet optimization for athletes/body builders

Most athletes and especially bodybuilders eat large amounts of protein to promote muscle growth. Protein intake may be in the order of 2 gram of protein per kg of body weight per day. The big question is how much of this protein is absorbed? With actual methods is estimated that about 50% of consumed protein appears in the blood.

To absorb protein, digestive proteases are crucial. It is commonly thought that mainly amino acids are absorbed. Our studies (among others) have shown that di- and tripeptides are absorbed, and we can measure absorption of these peptides. Appearance of these nutritional peptides is strongly dependent on availability of digestive enzymes.

The healthy body can produce enough enzymes for digestion of regular amount of protein (ca 1 g protein per kg body weight), however, the production of enzymes decreases with age. Additional digestive enzymes - especially proteases can improve peptide formation and affect training/performance effectiveness. Training and performance per se stimulate pancreas secretion and improve peptide (protein) absorption.

### How training improves protein (dietary peptide) absorption

**Test:** Six professional cyclists take part in the following:
1. The first day after regular breakfast at 0800 hours 6 cyclist (40 - 50 years af age) cycled 100 km (ca 3 hours without meal). Basal - 0 blood sample is withdrawn directly after training and meal reach in protein (1 hour consumption time) was offered.
2. The cyclists rest for 2-3 hours and 2nd blood sample is obtained.
3. Protein absorption is estimated with ninhydrin method of the invention as free and peptide bound amino acids.
4. In two days identical procedure is repeated with the same persons but instead of cycling they get free time and SPA (massage, water bath, sauna, light swimming, etc.).
5. The free amino acids and total (peptide bound) amino acid levels after SPA and free time was set up as base line and nominated as 100%. 5 hours after meal start the levels of free amino acids increased by 20% while total (peptide bound) amino acid level increased by 60%.
6. In the same persons after intensive cycling period amino acids level is decreased by 10% while total (peptide bound) amino acid level decreased by 30% as compared to baseline from free - SPA time. 2-3 hours after meal began the levels of free amino acids increase by 10% while total (peptide bound) amino acid level increased by 100% as compared to baseline in free time.

The results imply that intensive training depletes blood from peptide bound amino acids and protein from meal is absorbed in higher magnitude than after the resting time.

Results imply also that monitoring postprandial blood peptide level (peptide absorption) after meals can be a tool to estimate effectiveness of a meal, dietary supplements, training as well as requirements for digestive enzyme supplementation.

### Example 8: Protein absorption after bariatric surgery

Patients after bariatric surgery normally exhibit symptoms of exocrine pancreatic insufficiency.

### Test:

1. 10 patients undergo bariatric surgery (Roux-en-Y gastric bypass) and 6 month later undergo the meal tests.
2. First day before morning meal the baseline 0 sample is drawn, and another blood sample is drawn 2-3 hours later.
3. Six days later, identical tests are performed on the same persons but in addition to the meal they ingest 4 tablets of Creon 10 000 and 150 mg of pepsin.
4. Plasma level of free amino acids and total amino acids are measured with the ninhydrin method of the invention.
5. Level of free and total (peptide bound) amino acids in 0 (baseline) samples are similar in both tests and are nominated as 100%.
6. 2-3 hours after meal level of free amino acids in first test is increased by 10%, while peptide bound amino acids by 40%.
7. 2-3 hours after meal in second test when Creon 10 000 and pepsin are used, the free amino acids goes up by 25 % while peptide bound amino acids significantly rises by 100% or more.

In conclusion, results imply that gastric and pancreatic enzyme insufficiency can reduce protein absorption in form of peptides. The effects may be quantitated using the inventive method.

### Example 9: Optimizing diet for geriatric patients

Senior people constitute the fastest growing segment of the population. The elderly are at risk for malnutrition, thought to be caused by reduced food intake or impairment of the physiological capacity of the GI tract. Age-related changes are well known in organs such as liver, kidney and intestine. The pancreas, representing a metabolically active organ with uptake and breakdown of essential nutritional components, changes its morphology and function with age.

During childhood, the volume of the pancreas increases, reaching a plateau between 20 and 60 years, and declines thereafter. This decline involves the pancreatic parenchyma and is associated with decreased perfusion, fibrosis and atrophy. Because of these changes, pancreatic exocrine function is impaired in healthy older individuals without any gastrointestinal disease. Five per cent of people older than 70 years and ten per cent older than 80 years have EPI with a faecal elastase-1 levels below 200 µg g⁻¹ stool, and 5% have severe EPI with faecal elastase-1 levels below 100 µg g⁻¹stool. This may lead to maldigestion and malnutrition. Patients may have few symptoms, for example steatorrhea, diarrhoea, abdominal pain and weight loss. Malnutrition consists of deficits of fat-soluble vitamins and is affecting both patients with EPI and the elderly. Secondary consequences may include decreased bone mineral density and results from impaired absorption of protein and fat-soluble vitamin D due to impaired pancreatic exocrine function. The unanswered question is whether this age-related decrease in pancreatic function warrants therapy. Therapeutic intervention, which may consist of supplementation of pancreatic enzymes and/or vitamins in aged individuals with proven exocrine pancreas insufficiency, could contribute to healthy ageing.

During ageing, muscle and strength are progressively lost. This process of losing muscle with age is called sarcopenia. One of the underlying mechanisms of sarcopenia is a lower anabolic response to protein ingestion. However, it is not fully understood why older adults are less responsive to protein. In the study by Chapman et al. (2021) , authors combined data from all previous studies using intrinsically labelled protein. Intrinsically labelled protein is a special type of protein that was produced for research, that allowed authors to follow it throughout to body to measure its absorption. This allowed authors to make all kind of interesting comparisons, such as differences in protein digestion and absorption between young and older adults. For young adults, 60% of ingested protein ended up in the blood following digestion and absorption. For older adults, this was only 40%. Therefore, less of the ingested protein may become available for muscle tissue in older adults. This may suggest that older adults need to consume slightly more protein to compensate.

**In conclusion,** protein digestion and absorption into the circulation in elderly is in general low. It is not known how big portion of digestive protein is absorbed in form of tripeptides and dipeptides. This can be of importance for regeneration of the host protein especially in elderly. The complementary transport of dietary peptides from blood to particular tissues of intensive metabolism (muscle, brain, liver, pancreas, etc.) by PepT2 transporter is of importance to understand the role of absorbed dietary peptides.

### Test

1. 12 patients of age between 60 and 80 years agree to perform meal tests.
2. First day before morning meal the baseline 0 sample is taken and another blood sample is drawn 2-3 hours latter
3. Six days after identical test is performed on the same persons but with the additional intake of 4 tablets of Creon 10 000.
4. Plasma level of free amino acids and total amino acids is measured with described ninhydrin method.
5. Level of free and total (peptide bound) amino acids in 0 (baseline) samples are similar in both tests and nominated as 100%.
6. 2-3 hours after meal level of free amino acids in first test is increased by 50 % while peptide bound amino acids are increased by 20%.
7. 2-3 hours after meal in second test when Creon 10 000 is used as dietary supplements the free amino acids go up by 15 % while peptide bound amino acids significantly rise by 70%.

In conclusion, results imply that aging patients suffer from exocrine pancreatic insufficiency which can reduce protein absorption in form of peptides. The effects may be quantitated using the inventive method.

### Example 10: Children with growth retardation

It is postulated that growth retardation in children is related to the low level of nutritional peptide absorption. Meal test with protein of various origin in the mixed meal is used to assess the best absorbable protein and/or necessity of protease supplementation. Test is performed as previously described, with serial postprandial blood sample collections and determination of CF, CT and CP by ninhydrin method.

### Example 11: Farm animals

It is postulated that higher level of absorbed dietary dipeptides and tripeptide over amino acids during early life can predict better productivity of farm animals in the performance period. Protein or mixed meal test with serial OR single postprandial blood sampling using the inventive method is used for early identification of best individual farm animals in terms of growth and/or productivity.

### MATERIALS AND METHODS

### Animals

The experiment was performed on crossbred ((Polish Landrace × Yorkshire) × Hampshire)) pigs (*Sus scrofa domesticus*)*.* Experiment was performed on 6 pigs of both genders with body weight of 15+2.3 at the beginning of the study. The same pigs underwent pancreatic duct ligation surgery 3 weeks after arrival to animal facility. After confirmed development of EPI (exocrine pancreatic insufficiency) jugular vein catheters were inserted. The study was approved by the Second Local Ethics Committee for Animal Experimentation in Warsaw, Poland (approval no. WAW2/025/2022). All efforts were made to minimize animal suffering.

### Experiment design and study treatments

Pigs kept on standard diet were allocated to the study after 7days of adaptation.

By day 10 pigs were switched gradually to the high fat diet (HFD). Jugular catheters were implanted at day 11. At day 15 meal tolerance test (MTT) was performed on healthy pigs. On day 16 pigs were subjected to pancreatic duct ligation (PDL) surgery. EPI was allowed to be developed for 30 days. At day 47, identical MTT test was performed on EPI pigs. MTT was comprised of 160 g of HFD.

### Feeding

During the study pigs were fed with a high fat diet (HFD) (Kcynia, Morawski Plant, Poland) in an amount equivalent to 4% of their body weight daily with 1% (160 g) given at the morning meal (09:00-10:00 hr) and 3% (480 g) at the afternoon meal (17:00 - 18:00). Upon arrival pigs were fed a cereal-based, pelleted, standard diet being gradually changed by day 5 to HFD, containing 17.5% crude protein, 3.9% crude fibre, 20% crude fat, and 5.2% ash together with 5,000IE/kg vitamin A, 500 IE/kg vitamin D, 85 mg/kg vitamin E.

### Blood sampling

Blood samples were collected via the jugular vein catheter one hour prior to feeding and then at 60, 120, 180 and 240 minutes after feeding and transferred to BD Vacutainer^{®} glass Aprotinine K₃EDTA tubes (BD Diagnostics, New Jersey, USA). The blood samples were immediately placed on ice before they were centrifuged at 3000 × g for 15 minutes at 4°C, and plasma was separated and stored at -80°C until further analysis. The content of free, total, and peptide-derived amino acids in plasma samples was analysed by the below described method.

### Statistical analysis

Statistical analysis was performed on the data generated from this study using the Student's t-test with Welch correction for normally distributed datasets or Mann-Whitney test when data was not normally distributed. The data distribution was assessed using the Shapiro-Wilk normality test. Outliers within data sets were identified using the ROUT method of regression, using (Q=0.05%). All the analysis was cariied out using GraphPad Prism 10.0, San Diego, USA. Data was not corrected for multiple comparisons. Differences were considered significant if p≤0.05; differences were considered as a trend when *p*≤0.1; data with Gaussian distribution is expressed as mean ± standard deviation (± SD), data with non-Gaussian distribution is expressed as median ± intraquartile range (± IQR).

## Claims

1. An in vitro high throughput method for assessing protein absorption from the gut of a subject, comprising:
a. Providing a blood sample from the subject;
b. Substantially removing proteins having size over the size limit of 40 kDa from the sample to provide a deproteinized sample;
c. With a colorimetric ninhydrin assay determining the level of free amino acids in a subsample of the deproteinized sample, the level being designated CF;
d. Subjecting a subsample of the deproteinized sample to hydrolysis converting any remaining polypeptides and peptides to free amino acids, thus obtaining a hydrolyzed sample;
e. With a colorimetric ninhydrin assay determining the level of free amino acids in the hydrolyzed sample, the level being designated CT;
f. Optionally, calculating the level of peptide-derived amino-acids in the blood sample, the level being designated CP, wherein CP=CT-CF;
and
g. Assessing protein absorption from the gut of the subject based on the values of CF and CT, and optionally further using CP.

2. The method of any of the preceding claims, wherein an increased postprandial level of CF relative to postprandial level of CT or CP compared to a healthy reference is deemed indicative of protein malabsorption and/or exocrine pancreatic insufficiency.

3. The method of any of the preceding claims, wherein reduced postprandial level of CP compared to a healthy reference is deemed indicative of protein malabsorption and/or exocrine pancreatic insufficiency.

4. The method according to claim 1, further comprising assessing the quality of the dietary protein based on the results from assessment of protein absorption, wherein CP is calculated, and wherein:
a. when CP is less than 50% compared to CP of a reference dietary protein of satisfactory dietary quality, the quality of the test dietary protein is deemed poor; and
b. when CP is at least 50% compared to CP of a reference dietary protein of satisfactory dietary quality, the quality of the test dietary protein is deemed satisfactory.

5. The method according to claim 1, further comprising estimating the biological age of the subject based on the assessed level of protein absorption, optionally based on CP, and further independently optionally in combination with other physiological indicators of biological age,
wherein the biological age estimation includes comparing the assessed level of protein absorption to a reference value of similarly quantitated protein absorption in a group of reference subjects, said group being representative of subjects of a particular age or age interval.

6. The method according to claim 5, wherein the reference subjects are matched with the subject in terms of age, sex and/or health status.

7. The method according to claim 1, further comprising assessing drug absorption capacity of the subject based on the assessment of protein absorption.

8. The method according to claim 7, wherein the assessed drug absorption is peptide transporter (PepT1)-dependent absorption.

9. The method according to claim 7 or 8, comprising calculating the level of peptide-derived amino-acids in the blood sample, the level being designated CP, wherein CP=CT-CF, and wherein the drug uptake assessment is performed based on CP.

10. The method of any of the preceding claims, wherein the protein removal in step b results in at least 98% reduction of proteins having molecular weight over the size limit.

11. The method of any of the preceding claims, wherein the protein removal in step b involves ultrafiltration and/or wherein the hydrolysis step involves acid hydrolysis.

12. The method of any of the preceding claims, wherein the size limit is 30 kDa, preferably 10 kDa.

13. The method of any of the preceding claims, wherein method comprises calculating the level of peptide-derived amino-acids in the blood sample, the level being designated CP, wherein CP=CT-CF and wherein step of assessing protein absorption from the gut of the subject based on the values of CF, CT and CP.

14. The method of any of the preceding claims, wherein the blood sample is a plasma sample, a serum sample, a whole haemolyzed blood sample or isolated red blood cell sample.

15. The method any of the preceding claims, wherein the results from the assessment of protein absorption are used to evaluate the results of bariatric surgery, wherein the evaluation is preferably performed by comparing the values before and after surgery.

## Patentansprüche

1. In-vitro-Hochdurchsatzverfahren zur Beurteilung der Proteinabsorption aus dem Darm eines Subjekts, umfassend:
a. Bereitstellen einer Blutprobe des Subjekts;
b. Im Wesentlichen Entfernen von Proteinen mit einer Größe oberhalb der Größengrenze von 40 kDa aus der Probe, um eine deproteinisierte Probe bereitzustellen;
c. Bestimmen des Spiegels freier Aminosäuren in einer Teilprobe der deproteinisierten Probe mit einem kolorimetrischen Ninhydrin-Assay, wobei der Spiegel als CF bezeichnet wird;
d. Unterziehen einer Teilprobe der deproteinisierten Probe einer Hydrolyse, die etwaige verbleibende Polypeptide und Peptide in freie Aminosäuren umwandelt, wodurch eine hydrolysierte Probe erhalten wird;
e. Bestimmen des Spiegels freier Aminosäuren in der hydrolysierten Probe mit einem kolorimetrischen Ninhydrin-Assay, wobei der Spiegel als CT bezeichnet wird;
f. Optional Berechnen des Spiegels peptidabgeleiteter Aminosäuren in der Blutprobe, wobei der Spiegel als CP bezeichnet wird, wobei CP=CT-CF;
und
g. Beurteilen der Proteinabsorption aus dem Darm des Subjekts auf der Grundlage der Werte von CF und CT und optional zusätzlich unter Verwendung von CP.

2. Verfahren nach einem der vorhergehenden Ansprüche, wobei ein erhöhter postprandialer Spiegel von CF im Verhältnis zum postprandialen Spiegel von CT oder CP im Vergleich zu einer gesunden Referenz als Hinweis auf eine Proteinmalabsorption und/oder eine exokrine Pankreasinsuffizienz angesehen wird.

3. Verfahren nach einem der vorhergehenden Ansprüche, wobei ein verringerter postprandialer Spiegel von CP im Vergleich zu einer gesunden Referenz als Hinweis auf eine Proteinmalabsorption und/oder eine exokrine Pankreasinsuffizienz angesehen wird.

4. Verfahren nach Anspruch 1, ferner umfassend das Beurteilen der Qualität des Nahrungsproteins auf der Grundlage der Ergebnisse der Beurteilung der Proteinabsorption, wobei CP berechnet wird und wobei:
a. wenn CP im Vergleich zu CP eines Referenz-Nahrungsproteins mit zufriedenstellender ernährungsphysiologischer Qualität weniger als 50 % beträgt, die Qualität des Test-Nahrungsproteins als schlecht angesehen wird; und
b. wenn CP im Vergleich zu CP eines Referenz-Nahrungsproteins mit zufriedenstellender ernährungsphysiologischer Qualität mindestens 50 % beträgt, die Qualität des Test-Nahrungsproteins als zufriedenstellend angesehen wird.

5. Verfahren nach Anspruch 1, ferner umfassend das Schätzen des biologischen Alters des Subjekts auf der Grundlage des beurteilten Niveaus der Proteinabsorption, optional auf der Grundlage von CP, und ferner unabhängig davon optional in Kombination mit anderen physiologischen Indikatoren des biologischen Alters, wobei die Schätzung des biologischen Alters das Vergleichen des beurteilten Niveaus der Proteinabsorption mit einem Referenzwert einer auf ähnliche Weise quantifizierten Proteinabsorption in einer Gruppe von Referenzsubjekten umfasst, wobei die genannte Gruppe repräsentativ für Subjekte eines bestimmten Alters oder Altersintervalls ist.

6. Verfahren nach Anspruch 5, wobei die Referenzsubjekte hinsichtlich Alter, Geschlecht und/oder Gesundheitszustand auf das Subjekt abgestimmt sind.

7. Verfahren nach Anspruch 1, ferner umfassend das Beurteilen der Arzneimittelabsorptionskapazität des Subjekts auf der Grundlage der Beurteilung der Proteinabsorption.

8. Verfahren nach Anspruch 7, wobei die beurteilte Arzneimittelabsorption eine Peptidtransporter(PepT1)-abhängige Absorption ist.

9. Verfahren nach Anspruch 7 oder 8, umfassend das Berechnen des Spiegels peptidabgeleiteter Aminosäuren in der Blutprobe, wobei der Spiegel als CP bezeichnet wird, wobei CP=CT-CF, und wobei die Beurteilung der Arzneimittelaufnahme auf der Grundlage von CP durchgeführt wird.

10. Verfahren nach einem der vorhergehenden Ansprüche, wobei die Proteinentfernung in Schritt b zu einer Verringerung der Proteine mit einem Molekulargewicht oberhalb der Größengrenze um mindestens 98 % führt.

11. Verfahren nach einem der vorhergehenden Ansprüche, wobei die Proteinentfernung in Schritt b eine Ultrafiltration umfasst und/oder wobei der Hydrolyseschritt eine Säurehydrolyse umfasst.

12. Verfahren nach einem der vorhergehenden Ansprüche, wobei die Größengrenze 30 kDa, vorzugsweise 10 kDa, beträgt.

13. Verfahren nach einem der vorhergehenden Ansprüche, wobei das Verfahren das Berechnen des Spiegels peptidabgeleiteter Aminosäuren in der Blutprobe umfasst, wobei der Spiegel als CP bezeichnet wird, wobei CP=CT-CF, und wobei der Schritt des Beurteilens der Proteinabsorption aus dem Darm des Subjekts auf der Grundlage der Werte von CF, CT und CP.

14. Verfahren nach einem der vorhergehenden Ansprüche, wobei die Blutprobe eine Plasmaprobe, eine Serumprobe, eine hämolysierte Vollblutprobe oder eine Probe isolierter roter Blutkörperchen ist.

15. Verfahren nach einem der vorhergehenden Ansprüche, wobei die Ergebnisse der Beurteilung der Proteinabsorption verwendet werden, um die Ergebnisse einer bariatrischen Operation zu bewerten, wobei die Bewertung vorzugsweise durch Vergleichen der Werte vor und nach der Operation durchgeführt wird.

## Revendications

1. Procédé in vitro à haut débit pour évaluer l'absorption des protéines à partir de l'intestin d'un sujet, comprenant les étapes consistant à :
a. fournir un échantillon sanguin du sujet ;
b. éliminer sensiblement de l'échantillon les protéines ayant une taille supérieure à la limite de taille de 40 kDa pour fournir un échantillon déprotéinisé ;
c. au moyen d'un dosage colorimétrique à la ninhydrine, déterminer le niveau d'acides aminés libres dans un sous-échantillon de l'échantillon déprotéinisé, le niveau étant désigné par CF ;
d. soumettre un sous-échantillon de l'échantillon déprotéinisé à une hydrolyse convertissant les éventuels polypeptides et peptides restants en acides aminés libres, obtenant ainsi un échantillon hydrolysé ;
e. au moyen d'un dosage colorimétrique à la ninhydrine, déterminer le niveau d'acides aminés libres dans l'échantillon hydrolysé, le niveau étant désigné par CT ;
f. facultativement, calculer le niveau d'acides aminés dérivés de peptides dans l'échantillon sanguin, le niveau étant désigné par CP, avec CP=CT-CF ;
et
g. évaluer l'absorption des protéines à partir de l'intestin du sujet sur la base des valeurs de CF et de CT, et facultativement en utilisant en outre CP.

2. Procédé selon l'une quelconque des revendications précédentes, dans lequel un niveau postprandial accru de CF par rapport au niveau postprandial de CT ou de CP comparé à une référence saine est considéré comme indicatif d'une malabsorption des protéines et/ou d'une insuffisance pancréatique exocrine.

3. Procédé selon l'une quelconque des revendications précédentes, dans lequel un niveau postprandial réduit de CP comparé à une référence saine est considéré comme indicatif d'une malabsorption des protéines et/ou d'une insuffisance pancréatique exocrine.

4. Procédé selon la revendication 1, comprenant en outre l'évaluation de la qualité de la protéine alimentaire sur la base des résultats de l'évaluation de l'absorption des protéines, dans lequel CP est calculé, et dans lequel :
a. lorsque CP est inférieur à 50 % comparé au CP d'une protéine alimentaire de référence de qualité nutritionnelle satisfaisante, la qualité de la protéine alimentaire testée est considérée comme médiocre ; et
b. lorsque CP est d'au moins 50 % comparé au CP d'une protéine alimentaire de référence de qualité nutritionnelle satisfaisante, la qualité de la protéine alimentaire testée est considérée comme satisfaisante.

5. Procédé selon la revendication 1, comprenant en outre l'estimation de l'âge biologique du sujet sur la base du niveau d'absorption des protéines évalué, facultativement sur la base de CP, et en outre, indépendamment, facultativement en combinaison avec d'autres indicateurs physiologiques de l'âge biologique, dans lequel l'estimation de l'âge biologique comprend la comparaison du niveau d'absorption des protéines évalué avec une valeur de référence d'absorption des protéines quantifiée de manière similaire dans un groupe de sujets de référence, ledit groupe étant représentatif de sujets d'un âge ou d'une tranche d'âge particuliers.

6. Procédé selon la revendication 5, dans lequel les sujets de référence sont appariés au sujet en termes d'âge, de sexe et/ou d'état de santé.

7. Procédé selon la revendication 1, comprenant en outre l'évaluation de la capacité d'absorption de médicaments du sujet sur la base de l'évaluation de l'absorption des protéines.

8. Procédé selon la revendication 7, dans lequel l'absorption de médicaments évaluée est une absorption dépendante du transporteur de peptides (PepT1).

9. Procédé selon la revendication 7 ou 8, comprenant le calcul du niveau d'acides aminés dérivés de peptides dans l'échantillon sanguin, le niveau étant désigné par CP, avec CP=CT-CF, et dans lequel l'évaluation de la captation de médicaments est réalisée sur la base de CP.

10. Procédé selon l'une quelconque des revendications précédentes, dans lequel l'élimination des protéines à l'étape b entraîne une réduction d'au moins 98 % des protéines ayant un poids moléculaire supérieur à la limite de taille.

11. Procédé selon l'une quelconque des revendications précédentes, dans lequel l'élimination des protéines à l'étape b implique une ultrafiltration et/ou dans lequel l'étape d'hydrolyse implique une hydrolyse acide.

12. Procédé selon l'une quelconque des revendications précédentes, dans lequel la limite de taille est de 30 kDa, de préférence de 10 kDa.

13. Procédé selon l'une quelconque des revendications précédentes, dans lequel le procédé comprend le calcul du niveau d'acides aminés dérivés de peptides dans l'échantillon sanguin, le niveau étant désigné par CP, avec CP=CT-CF, et dans lequel l'étape d'évaluation de l'absorption des protéines à partir de l'intestin du sujet sur la base des valeurs de CF, CT et CP.

14. Procédé selon l'une quelconque des revendications précédentes, dans lequel l'échantillon sanguin est un échantillon de plasma, un échantillon de sérum, un échantillon de sang total hémolysé ou un échantillon de globules rouges isolés.

15. Procédé selon l'une quelconque des revendications précédentes, dans lequel les résultats de l'évaluation de l'absorption des protéines sont utilisés pour apprécier les résultats d'une chirurgie bariatrique, dans lequel l'appréciation est de préférence réalisée en comparant les valeurs avant et après la chirurgie.
